(19) Europäisches Patentamt
European Patent Office
Office européen des brevets

(11) EP 4 799 648 A1

(12) **EUROPEAN PATENT APPLICATION**
published in accordance with Art. 153(4) EPC

(43) Date of publication:
**02.09.2026 Bulletin 2026/36**

(21) Application number: **24881719.9**

(22) Date of filing: **25.10.2024**

(51) International Patent Classification (IPC):
***A61K 47/68*** (2017.01) ***A61K 39/395*** (2006.01)
***A61K 31/4745*** (2006.01) ***C07K 16/28*** (2006.01)
***A61P 35/00*** (2006.01)

(52) Cooperative Patent Classification (CPC):
**A61K 31/4745; A61K 39/395; A61K 47/68; A61P 35/00; C07K 16/28**

(86) International application number:
**PCT/CN2024/127218**

(87) International publication number:
**WO 2025/087358 (01.05.2025 Gazette 2025/18)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **27.10.2023 CN 202311412815**

(71) Applicants:
- **Shanghai Hansoh Biomedical Company Limited Pudong New Area, Shanghai 201203 (CN)**
- **Changzhou Hansoh Pharmaceutical Company Limited Changzhou, Jiangsu 213001 (CN)**

(72) Inventors:
- **WEI, Hongying Shanghai 201203 (CN)**
- **HUANG, Qiqi Shanghai 201203 (CN)**
- **HUANG, Ying Shanghai 201203 (CN)**
- **LI, Chuan Shanghai 201203 (CN)**
- **DONG, Yiwei Shanghai 201203 (CN)**

(74) Representative: **Hanby, Abigail Rose**
**GSK**
**Legal & Compliance - Global Patents**
**79 New Oxford Street**
**London WC1A 1DG (GB)**

(54) **METHOD FOR TREATING CANCER USING ANTIBODY-DRUG CONJUGATE**

(57) The present invention relates to a method for treating a cancer using an antibody-drug conjugate. Specifically, the present invention provides a method for treating a cancer using an antibody-drug conjugate, a pharmaceutically acceptable salt thereof, a stereoisomer thereof, or a metabolite thereof, or a solvate of any one of the foregoing, and use of the antibody-drug conjugate in preparing a medicament for preventing and/or treating a cancer. The method features improved safety and prospective anti-tumor activity in a number of tumors, thus satisfying medical requirements.

Phase IIa
Screening period
Treatment period
The planned total enrollment is approximately 80-120 patients
Follow-up period
Study population:
Advanced solid tumors (mainly esophageal cancer)
At least 1 target lesion
ECOG PS 0-1
Expected survival ≥ 12 weeks
Good organ function
Cohort 1: Patients with unresectable locally advanced, recurrent, or metastatic esophageal squamous cell carcinoma who have progressed after at least one prior line of therapy or are intolerant to such therapy
Anti-B7-H3 antibody-drug conjugate, 10 mg/kg, administered intravenously Q3W (approximately 40 patients)
Cohort 2: Patients with unresectable locally advanced, recurrent, or metastatic esophageal adenocarcinoma or adenocarcinoma of the esophagogastric junction who have progressed after at least one prior line of therapy or are intolerant to such therapy
Anti-B7-H3 antibody-drug conjugate, 10 mg/kg, administered intravenously Q3W (approximately 20 patients)
Cohort 3: Patients with other advanced solid tumors for whom no standard treatment is available or who have failed standard therapy
Anti-B7-H3 antibody-drug conjugate, 10 mg/kg, administered intravenously Q3W (approximately 20 patients)
Primary endpoint:
ORR as assessed by the investigator according to RECIST 1.1
Secondary endpoints:
Efficacy (DCR, RoR, PFS, and OS)
Safety
PK characteristics
Immunogenicity
Exploratory endpoints:
Correlation between B7-H3 expression and efficacy, etc.
Phase IIb
Screening period
Treatment period
The planned total enrollment is approximately 100 patients
Follow-up period
Study population:
Esophageal squamous cell carcinoma
At least 1 target lesion
ECOG PS 0-1
Expected survival ≥ 12 weeks
Adequate organ function
Patients with unresectable locally advanced, recurrent, or metastatic esophageal squamous cell carcinoma who have progressed after or are intolerant to at least first-line treatment
Anti-B7-H3 antibody-drug conjugate, 10 mg/kg, administered intravenously Q3W (the estimated total enrollment is approximately 100 patients; the final sample size will be determined by statistical calculations before the Phase II trial)
Primary endpoint:
ORR as assessed by the investigator according to RECIST 1.1
Secondary endpoints:
Efficacy (DCR, RoR, PFS, and OS)
Safety
PK characteristics
Immunogenicity
Exploratory endpoints:
Correlation between B7-H3 expression and efficacy, etc.
Figure 2

EP 4 799 648 A1

## Description

### Technical Field

The present application belongs to the field of medicine and relates to a method for treating cancer using an antibody-drug conjugate. Specifically, the present invention provides a method for treating cancer with an antibody-drug conjugate, and use of the antibody-drug conjugate in the preparation of a medicine for treating cancer.

### Background Art

Antibody-drug conjugates (ADCs) are a class of drugs in which monoclonal antibodies and small-molecule cytotoxins are conjugated by chemical linkers. Through the guiding effect of the monoclonal antibodies, the conjugates bind to antigens on the surface of target cells and deliver the small-molecule cytotoxins into the target cells, thereby killing the target cells. ADCs possess both the high targeting specificity of antibodies and the potent cytotoxicity of cytotoxic agents, and have become a major focus of research and development in targeted cancer therapy.

The mechanism of action of an ADC includes: 1) toxin effect: this is the main mechanism by which an ADC exerts an antitumor effect, wherein through the specific binding of the antibody portion of the ADC to antigens on the surface of target cells, the ADC-antigen complex enters the target cells through endocytosis, is degraded in lysosomes to release the small-molecule cytotoxic drug, and finally the cytotoxic payload induces apoptosis through pathways such as damaging DNA or acting on microtubule proteins; 2) bystander effect: after the small-molecule cytotoxic drug kills the target cells, it penetrates into the extracellular space and produces a killing effect on surrounding cells; 3) antibody-dependent cell-mediated cytotoxicity (ADCC) and complement-dependent cytotoxicity (CDC): the intrinsic ADCC and CDC activities of the antibody component of the ADC also contribute to its cytotoxic effect.

Commonly used ADC targets include leukocyte surface antigens expressed in hematologic tumors, such as CD22, CD30, and CD33 as well as HER2, TROP2, and the like expressed on the surface of solid tumors. As of June 20, 2022, a total of 14 ADC products had been approved or marketed globally, and 5 had been marketed in China (with indications covering breast cancer, lymphoma, acute lymphoblastic leukemia, and gastric cancer), and the strong efficacy and low toxicity of ADCs have brought new hope for cancer treatment.

B7-H3 (B7 homolog 3 protein), also known as CD276, is an important immune checkpoint molecule of the B7-CD28 family. It is a B7 family member identified by Chapoval et al. [1] in 2001 in a human dendritic cell cDNA library, and plays an important role in tumor growth and immune regulation. B7-H3 is a type I transmembrane glycoprotein composed of 316 amino acids, with a molecular weight of 45-66 kDa, and the structure comprises 1 amino-terminal signal peptide, 1 extracellular immunoglobulin-like variable region (IgV) and constant region (IgC), 1 transmembrane region, and 1 cytoplasmic tail region containing 45 amino acids. B7-H3 has two subtypes: 2Ig-B7-H3 and 4Ig-B7-H3. 2Ig-B7-H3 is expressed in mouse and human cells, and it has an extracellular IgV-IgC structure; 4Ig-B7-H3 is expressed only in human cells, consists of a tandemly repeated IgV-IgC-IgV-IgC structure, is the predominant form of human B7-H3, and is located on chromosome 15 [2]. B7-H3 exists in two forms, a membrane protein form and a soluble form, wherein the soluble B7-H3 is derived from the membrane protein by metalloprotease cleavage [3], and its concentration in human serum is measured in nanograms per milliliter.

The receptor for human B7-H3 has not yet been identified. Research reports [4] show that B7-H3 may play a dual role in the immune system. On the one hand, B7-H3 has a costimulatory effect on CD4+ and CD8+ T cells, induces cellular immunity, and increases the production of interferon-$\gamma$ and interleukin-12; on the other hand, B7-H3 can exert an inhibitory effect on T cells, negatively regulate helper T-cell Th1/Th2-mediated immune responses and the accumulation of regulatory T cells; it can also inhibit NK cell-mediated cell lysis, contributing to the immune escape of tumor cells. In addition, B7-H3 can regulate the growth, migration, and invasion of tumor cells through signaling pathways such as PI3K/AKT/STAT3, JAK2/STAT3, and NF-$\kappa$B.

B7-H3 mRNA is widely expressed in almost all human immune and non-immune tissues and organs, but the expression of its protein in normal tissues is very limited, and it has certain low-level expression in the breast, prostate, testis, liver, lung, placenta, and some lymphoid organs. The difference between the widespread expression of B7-H3 mRNA and the limited distribution of the protein indicates that there is a complex post-transcriptional regulatory mechanism between the two. At present, an increasing number of studies have reported that B7-H3 is aberrantly overexpressed in a variety of tumor cells/tissues and in the tumor vascular system [5-12]. In addition, many studies have also shown that the high expression of B7-H3 is closely related to the biological characteristics of tumors, disease progression, and patient prognosis.

At present, a total of 5 ADC drugs targeting B7-H3 have been in clinical studies globally. In addition to the B7-H3-targeting ADC of the present invention, there are also MacroGenics' MGC018, AbbVie's ABBV-155 (Mirzotamab Clezutoclax), Daiichi Sankyo's DS-7300a, and Bio-Thera's BAT8009, all of which are in the early clinical research stage. By targeting the B7-H3 protein expressed by tumor cells, B7-H3 ADC drugs release small-molecule cytotoxins in

lysosomes, thereby inducing cell apoptosis; owing to the bystander effect, the drug may still be effective in patients whose tumors express low levels of B7-H3. Published research data show that therapeutic responses have been observed for B7-H3 ADC drugs in metastatic castration-resistant prostate cancer (mCRPC), non-small cell lung cancer (NSCLC), small cell lung cancer (SCLC), esophageal squamous cell carcinoma (ESCC), and head and neck squamous cell carcinoma (HNSCC).

Esophageal cancer is currently the eighth most common malignant tumor worldwide and ranks sixth among causes of cancer death. In 2020, globally there were about 604,000 new cases of esophageal cancer, and about 544,000 deaths from esophageal cancer. In 2020, in China there were about 324,000 new cases of esophageal cancer, and about 301,000 deaths from esophageal cancer. China is the country with the largest number of newly diagnosed esophageal cancer cases in the world, esophageal cancer ranks sixth in cancer incidence in China and fourth in mortality [13]. Globally, the predominant histological subtype of esophageal cancer is squamous cell carcinoma, accounting for about 85%. Esophageal squamous cell carcinoma in the Chinese population accounts for more than 90% of all esophageal cancers, while the proportion of ESCC in the North American population is about 30% [14]. Esophageal cancer has a relatively poor prognosis, with a 5-year survival rate of less than 20% [15].

About 70% of esophageal cancer patients are already at a locally advanced stage or an advanced stage with distant metastasis at the time of diagnosis, and cannot undergo surgical resection. For the treatment of unresectable locally advanced esophageal cancer, the 2022 CSCO Guidelines for the Diagnosis and Treatment of Esophageal Cancer recommend radical concurrent chemoradiotherapy or systemic drug therapy + radiotherapy. For metastatic esophageal squamous cell carcinoma or HER-2-negative esophageal adenocarcinoma, the CSCO Guidelines recommend platinum-containing chemotherapy ± immune checkpoint inhibitors for first-line standard treatment, with a median OS of 8.6-17 months and a median PFS of 4.9-8.3 months [16-19]; for HER-2-positive metastatic esophageal adenocarcinoma, the first-line standard treatment is anti-HER-2 therapy combined with chemotherapy, with a median OS of 11.1-13.8 months and a median PFS of 5.5-6.7 months. After progression on first-line treatment, options for subsequent-line treatment are extremely limited, mainly immune checkpoint inhibitors or chemotherapy. Single-drug immunotherapy recommended by the 2022 CSCO Guidelines, such as pembrolizumab, camrelizumab (for esophageal squamous cell carcinoma), or nivolumab (for esophageal adenocarcinoma), can be used as a second-line or third-line treatment option for advanced esophageal cancer, with a median OS of 8.3-10.9 months, a median PFS of only 1.7-2.6 months, and an ORR of 13.1%-20.2% [20-21], showing limited overall benefit. At present, there remains a substantial unmet clinical need in esophageal cancer, and new antitumor drugs are urgently needed.

## Summary of the Invention

The present disclosure provides a method for preventing and/or treating cancer using an antibody-drug conjugate, comprising administering to a patient a therapeutically effective amount of the antibody-drug conjugate, a pharmaceutically acceptable salt, stereoisomer, or metabolite thereof, or a solvate of any of the foregoing, wherein the structure of the antibody-drug conjugate is as shown in formula (I):

In the formula, n is a non-zero integer or decimal number from 1 to 10, preferably a decimal or integer between 1 and 8, preferably a decimal or integer between 2 and 8, more preferably 3 to 8, and may be an integer or may be a decimal.

Pc is an anti-B7H3 antibody or an antigen-binding fragment thereof.

In some embodiments, the anti-B7H3 antibody or the antigen-binding fragment thereof comprises: heavy-chain HCDR1, HCDR2, and HCDR3, respectively as shown in SEQ ID NOs: 01, 02, and 03, and light-chain LCDR1, LCDR2, and LCDR3, respectively having the amino acid sequences set forth in SEQ ID NOs: 04, 05, and 06.

In the present invention, the amino acid sequences of the CDRs listed above are all shown according to the Kabat definition rules. However, it is well known to those skilled in the art that antibody CDRs can be defined by various methods in this field. Although the scope of protection claimed by the present invention is based on the sequences shown according to the Kabat definition rules, corresponding amino acid sequences under other CDR definition rules should also fall within the scope of protection of the present invention.

The CDR sequences mentioned above are shown in the following table:

Table 1 CDR sequences of each heavy chain and light chain

| HCDR1 | SSAMH SEQ ID NO: 01 | LCDR1 | GLSSGSVSTSHYPS SEQ ID NO: 04 |
|---|---|---|---|
| HCDR2 | VISYDGSNKYYVDSVKGS EQ ID NO: 02 | LCDR2 | NTNTRSS SEQ ID NO: 05 |
| HCDR3 | SARLYASFDY | LCDR3 | AIHVDRDIWV |
| | SEQ ID NO: 03 | | SEQ ID NO: 06 |
| Note: The CDR sequences are defined according to the Kabat definition rules. | | | |

Preferably, the anti-B7H3 antibody or the antigen-binding fragment thereof is selected from a humanized antibody or a fragment thereof.

In some optional embodiments, the anti-B7H3 antibody or the antigen-binding fragment thereof in the present application is selected from the group consisting of antibody fragments of Fab, Fab'-SH, Fv, scFv, and (Fab')$_2$ fragments.

In some optional embodiments, the anti-B7H3 antibody or the antigen-binding fragment thereof in the present application comprises a heavy-chain constant region of the human IgG1, IgG2, IgG3, or IgG4 isotype, preferably comprises a heavy-chain constant region of the IgG1 or IgG4 isotype.

In some other optional embodiments, the anti-B7H3 antibody or antigen-binding fragment thereof comprises a light-chain constant region of κ or λ.

Further preferably, the heavy-chain variable region sequence of the anti-B7H3 antibody or antigen-binding fragment thereof is the sequence as shown in SEQ ID NO: 07 or a variant thereof, and the light-chain variable region sequence is the sequence as shown in SEQ ID NO: 08 or a variant thereof.

The sequences of the heavy-chain and light-chain variable regions of the foregoing anti-B7H3 antibody or antigen-binding fragment thereof are as follows:

Heavy-chain variable region sequence

*QVQLVQSGGGVVQPGTSLRLSCAASGFIFS*SSAMH*WVRQAPGKGLEWVA*VISYDGSNKY YVDSVKG*RFTISRDNSKNTLYLQMNSLRAEDTAVYYCAR*SARLYASFDY*WGQGALVTVSS*

SEQ ID NO: 07

Light-chain variable region sequence

*DTVVTQEPSFSVSPGGTVTLTC*GLSSGSVSTSHYPS*WYQQTPGQAPRMLIY*NTNTRSS*GV PDRFSGSILGNKAALTITGAQADDESDYYC*AIHVDRDIWV*FGGGTKLTVL*

SEQ ID NO: 08

Note: the order is FR1-CDR1-FR2-CDR2-FR3-CDR3-FR4, and in the sequences, italicized is FR sequences, and underlined is CDR sequences, wherein the CDR sequences are derived from the Kabat definition rules.

Further, preferably, the heavy-chain sequence of the anti-B7H3 antibody or antigen-binding fragment thereof is the sequence as shown in SEQ ID NO: 09 or a variant thereof, and the light-chain sequence is the sequence as shown in SEQ ID NO: 10 or a variant thereof.

The sequences of the heavy chain and light chain of the foregoing anti-B7H3 antibody or antigen-binding fragment thereof are as follows:

Heavy-chain (IgG1) amino acid sequence: (SEQ ID NO: 09)

QVQLVQSGGGVVQPGTSLRLSCAASGFIFSSSAMHWVRQAPGKGLEWVAVISYD GSNKYYVDSVKGRFTISRDNSKNTLYLQMNSLRAEDTAVYYCARSARLYASFDYWG QGALVTVSSASTKGPSVFPLAPSSKSTSGGTAALGCLVKDYFPEPVTVSWNSGALTSG VHTFPAVLQSSGLYSLSSVVTVPSSSLGTQTYICNVNHKPSNTKVDKKVEPKSCDKTH TCPPCPAPELLGGPSVFLFPPKPKDTLMISRTPEVTCVVVDVSHEDPEVKFNWYVDGV EVHNAKTKPREEQYNSTYRVVSVLTVLHQDWLNGKEYKCKVSNKALPAPIEKTISKA KGQPREPQVYTLPPSRDELTKNQVSLTCLVKGFYPSDIAVEWESNGQPENNYKTTPPV LDSDGSFFLYSKLTVDKSRWQQGNVFSCSVMHEALHNHYTQKSLSLSPGK

Light-chain (λ) amino acid sequence: (SEQ ID NO: 10)

DTVVTQEPSFSVSPGGTVTLTCGLSSGSVSTSHYPSWYQQTPGQAPRMLIYNTNT RSSGVPDRFSGSILGNKAALTITGAQADDESDYYCAIHVDRDIWVFGGGTKLTVLGQP KANPTVTLFPPSSEELQANKATLVCLISDFYPGAVTVAWKADGSPVKAGVETTKPSKQ SNNKYAASSYLSLTPEQWKSHRSYSCQVTHEGSTVEKTVAPTEC

In some embodiments, the administration route of the antibody-drug conjugate, pharmaceutically acceptable salt, stereoisomer, or metabolite thereof, or a solvate of any of the foregoing, is oral administration, parenteral administration, or transdermal administration, wherein the parenteral administration mode is selected from intravenous injection, intravenous infusion, subcutaneous injection, and intramuscular injection, preferably intravenous infusion.

In some embodiments, the antibody-drug conjugate, pharmaceutically acceptable salt, stereoisomer, or metabolite thereof, or a solvate of any of the foregoing, is administered to a patient by intravenous infusion once every week, once every two weeks, once every three weeks, once every four weeks, or once every six weeks.

Preferably, the antibody-drug conjugate, pharmaceutically acceptable salt, stereoisomer, or metabolite thereof, or a solvate of any of the foregoing, is administered to a patient by intravenous infusion once every three weeks.

In some embodiments, the administered dose of the antibody-drug conjugate, pharmaceutically acceptable salt, stereoisomer, or metabolite thereof, or a solvate of any of the foregoing, is 0.1 mg/kg to 20 mg/kg, preferably 0.5 mg/kg to 18 mg/kg, more preferably 1 mg/kg to 16 mg/kg, and still more preferably 4 mg/kg to 12 mg/kg.

In some embodiments, the administered dose of the antibody-drug conjugate, pharmaceutically acceptable salt, stereoisomer, or metabolite thereof, or a solvate of any of the foregoing, is selected from 4.0 mg/kg, 4.1 mg/kg, 4.2 mg/kg, 4.3 mg/kg, 4.4 mg/kg, 4.5 mg/kg, 4.6 mg/kg, 4.7 mg/kg, 4.8 mg/kg, 4.9 mg/kg, 5 mg/kg, 5.1 mg/kg, 5.2 mg/kg, 5.3 mg/kg, 5.4 mg/kg, 5.5 mg/kg, 5.6 mg/kg, 5.7 mg/kg, 5.8 mg/kg, 5.9 mg/kg, 6.0 mg/kg, 6.1 mg/kg, 6.2 mg/kg, 6.3 mg/kg, 6.4 mg/kg, 6.5 mg/kg, 6.6 mg/kg, 6.7 mg/kg, 6.8 mg/kg, 6.9 mg/kg, 7 mg/kg, 7.1 mg/kg, 7.2 mg/kg, 7.3 mg/kg, 7.4 mg/kg, 7.5 mg/kg, 7.6 mg/kg, 7.7 mg/kg, 7.8 mg/kg, 7.9 mg/kg, 8 mg/kg, 8.1 mg/kg, 8.2 mg/kg, 8.3 mg/kg, 8.4 mg/kg, 8.5 mg/kg, 8.6 mg/kg, 8.7 mg/kg, 8.8 mg/kg, 8.9 mg/kg, 9 mg/kg, 9.1 mg/kg, 9.2 mg/kg, 9.3 mg/kg, 9.4 mg/kg, 9.5 mg/kg, 9.6 mg/kg, 9.7 mg/kg, 9.8 mg/kg, 9.9 mg/kg, 10 mg/kg, 10.1 mg/kg, 10.2 mg/kg, 10.3 mg/kg, 10.4 mg/kg, 10.5 mg/kg, 10.6 mg/kg, 10.7 mg/kg, 10.8 mg/kg, 10.9 mg/kg, 11 mg/kg, 11.1 mg/kg, 11.2 mg/kg, 11.3 mg/kg, 11.4 mg/kg, 11.5 mg/kg, 11.6 mg/kg, 11.7 mg/kg, 11.8 mg/kg, 11.9 mg/kg, or 12 mg/kg.

In some embodiments, 8 mg/kg or 10 mg/kg of the antibody-drug conjugate, pharmaceutically acceptable salt, stereoisomer, or metabolite thereof, or a solvate of any of the foregoing, is administered to a patient by intravenous infusion once every three weeks.

In some embodiments, the cancer is selected from breast cancer, ovarian cancer, cervical cancer, lung cancer, uterine cancer, prostate cancer, kidney cancer, urethral cancer, bladder cancer, liver cancer, gastric cancer, salivary gland cancer, esophageal cancer, melanoma, glioma, neuroblastoma, sarcoma, head and neck cancer, colorectal cancer, leukemia, bone cancer, skin cancer, thyroid cancer, pancreatic cancer, and lymphoma; the prostate cancer is preferably castration-resistant prostate cancer, the head and neck cancer is preferably head and neck squamous cell carcinoma, and the esophageal cancer is preferably esophageal squamous cell carcinoma, esophageal adenocarcinoma, or adenocarcinoma of the esophagogastric junction.

In some embodiments, the patient is a pathologically confirmed patient with unresectable locally advanced, recurrent, or metastatic cancer, or another advanced solid tumor patient, preferably a patient with unresectable locally advanced, recurrent, or metastatic cancer who has progressed after or is intolerant to at least first-line treatment.

In some embodiments, the patient is a patient with unresectable locally advanced, recurrent, or metastatic esophageal squamous cell carcinoma who has progressed after or is intolerant to at least first-line treatment; and/or a patient with unresectable locally advanced, recurrent, or metastatic esophageal adenocarcinoma or adenocarcinoma of

the esophagogastric junction who has progressed after or is intolerant to at least first-line treatment.

In some embodiments, the unresectable locally advanced or recurrent esophageal squamous cell carcinoma patient who has progressed after or is intolerant to at least first-line treatment is a patient with unresectable locally advanced or locally recurrent esophageal squamous cell carcinoma who needs to have received, at the locally advanced or recurrent stage, at least (radical) concurrent chemoradiotherapy or sequential chemoradiotherapy; the metastatic esophageal squamous cell carcinoma patient who has progressed after or is intolerant to at least first-line treatment is a metastatic esophageal squamous cell carcinoma patient who has received at least platinum-containing chemotherapy.

In some embodiments, the patient with unresectable locally advanced or locally recurrent esophageal adenocarcinoma or adenocarcinoma of the esophagogastric junction is required to have received at least (radical) concurrent chemoradiotherapy or sequential chemoradiotherapy.

In some embodiments, the metastatic esophageal adenocarcinoma or adenocarcinoma of the esophagogastric junction patient is a HER-2-positive metastatic esophageal adenocarcinoma or adenocarcinoma of the esophagogastric junction patient, or a HER-2-negative metastatic esophageal adenocarcinoma or adenocarcinoma of the esophagogastric junction patient.

In some embodiments, the HER-2-positive metastatic esophageal adenocarcinoma or adenocarcinoma of the esophagogastric junction patient has received at least anti-HER-2 therapy combined with platinum-containing chemotherapy; the HER-2-negative metastatic esophageal adenocarcinoma or adenocarcinoma of the esophagogastric junction patient has received at least platinum-containing chemotherapy.

In some embodiments, the other advanced solid tumor patient is another advanced solid tumor patient for whom there is no standard treatment regimen or who has failed standard treatment.

Further, the method comprises selecting the patient based on an elevated level of a biomarker related to cancer.

In some embodiments, evaluation of the therapeutic effect comprises detecting the concentration of a biomarker in a patient sample, and preferably, the biomarker is selected from soluble B7-H3 and PD-L1.

Further, by determining the soluble B7-H3 concentration detected from patient blood samples collected during the screening period, during treatment, and at the end-of-treatment visit and/or collected for determining the PD-L1 protein expression in patient tumor tissue samples, the relationship between the biomarker and the efficacy of the antibody-drug conjugate is evaluated.

Another aspect of the present invention further provides use of an antibody-drug conjugate, a pharmaceutically acceptable salt, stereoisomer, or metabolite thereof, or a solvate of any of the foregoing, in the preparation of a medicine for treating cancer, wherein the antibody-drug conjugate is as shown in formula (I) above,
In the formula:

n is 1 to 10, preferably 2 to 8, more preferably 3 to 8, and n is a decimal or an integer;
Pc is an anti-B7H3 antibody or an antigen-binding fragment thereof;

The cancer is selected from breast cancer, ovarian cancer, cervical cancer, lung cancer, uterine cancer, prostate cancer, kidney cancer, urethral cancer, bladder cancer, liver cancer, gastric cancer, salivary gland cancer, esophageal cancer, melanoma, glioma, neuroblastoma, sarcoma, head and neck cancer, colorectal cancer, leukemia, bone cancer, skin cancer, thyroid cancer, pancreatic cancer, and lymphoma; the prostate cancer is preferably castration-resistant prostate cancer, the head and neck cancer is preferably head and neck squamous cell carcinoma, and the esophageal cancer is preferably esophageal squamous cell carcinoma, esophageal adenocarcinoma, or adenocarcinoma of the esophagogastric junction.

Further, the anti-B7H3 antibody or an antigen-binding fragment thereof comprises: heavy-chain HCDR1, HCDR2, and HCDR3 respectively as shown in the amino acid sequences of SEQ ID NO: 01, 02, and 03, and light-chain LCDR1, LCDR2, and LCDR3 respectively as shown in the amino acid sequences of SEQ ID NO: 04, 05, and 06.

In some embodiments, the anti-B7H3 antibody or an antigen-binding fragment thereof is selected from a humanized antibody or a fragment thereof.

In some embodiments, the anti-B7H3 antibody or an antigen-binding fragment thereof comprises a heavy-chain constant region of the human IgG1, IgG2, IgG3, or IgG4 isotype, and comprises a light-chain constant region of κ or λ; preferably, the anti-B7H3 antibody or antigen-binding fragment thereof comprises a heavy-chain constant region of the IgG1 or IgG4 isotype.

Preferably, the heavy-chain variable region sequence of the anti-B7H3 antibody or an antigen-binding fragment thereof is as shown in SEQ ID NO: 07, and the light-chain variable region sequence is as shown in SEQ ID NO: 08.

Further, the heavy-chain sequence of the anti-B7H3 antibody or an antigen-binding fragment thereof is as shown in SEQ ID NO: 09, and the light-chain sequence is as shown in SEQ ID NO: 10.

In some embodiments, the prevention and/or treatment comprises administering to a patient a therapeutically effective amount of the anti-B7H3 antibody-drug conjugate of formula (I), a pharmaceutically acceptable salt, stereoisomer, or metabolite thereof, or a solvate of any of the foregoing, wherein the route of administration is oral administration,

parenteral administration, or transdermal administration, and the parenteral administration mode is selected from intravenous injection, intravenous infusion, subcutaneous injection, and intramuscular injection, preferably intravenous infusion.

In some embodiments, the antibody-drug conjugate is administered to a patient by intravenous infusion once every week, once every two weeks, once every three weeks, once every four weeks, or once every six weeks, preferably once every three weeks.

In some embodiments, the prevention and/or treatment comprises administering to a patient a therapeutically effective amount of the anti-B7-H3 antibody-drug conjugate of formula (I), a pharmaceutically acceptable salt, stereoisomer, or metabolite thereof, or a solvate of any of the foregoing, at a dose of 0.5-18 mg/kg, preferably at a dose of 1-16 mg/kg, more preferably at a dose of 4-12 mg/kg.

In some embodiments, the prevention and/or treatment comprises administering to a patient a therapeutically effective amount of the anti-B7-H3 antibody-drug conjugate of formula (I), a pharmaceutically acceptable salt, stereoisomer, or metabolite thereof, or a solvate of any of the foregoing, wherein the administered dose is selected from 4.0 mg/kg, 4.1 mg/kg, 4.2 mg/kg, 4.3 mg/kg, 4.4 mg/kg, 4.5 mg/kg, 4.6 mg/kg, 4.7 mg/kg, 4.8 mg/kg, 4.9 mg/kg, 5 mg/kg, 5.1 mg/kg, 5.2 mg/kg, 5.3 mg/kg, 5.4 mg/kg, 5.5 mg/kg, 5.6 mg/kg, 5.7 mg/kg, 5.8 mg/kg, 5.9 mg/kg, 6.0 mg/kg, 6.1 mg/kg, 6.2 mg/kg, 6.3 mg/kg, 6.4 mg/kg, 6.5 mg/kg, 6.6 mg/kg, 6.7 mg/kg, 6.8 mg/kg, 6.9 mg/kg, 7 mg/kg, 7.1 mg/kg, 7.2 mg/kg, 7.3 mg/kg, 7.4 mg/kg, 7.5 mg/kg, 7.6 mg/kg, 7.7 mg/kg, 7.8 mg/kg, 7.9 mg/kg, 8 mg/kg, 8.1 mg/kg, 8.2 mg/kg, 8.3 mg/kg, 8.4 mg/kg, 8.5 mg/kg, 8.6 mg/kg, 8.7 mg/kg, 8.8 mg/kg, 8.9 mg/kg, 9 mg/kg, 9.1 mg/kg, 9.2 mg/kg, 9.3 mg/kg, 9.4 mg/kg, 9.5 mg/kg, 9.6 mg/kg, 9.7 mg/kg, 9.8 mg/kg, 9.9 mg/kg, 10 mg/kg, 10.1 mg/kg, 10.2 mg/kg, 10.3 mg/kg, 10.4 mg/kg, 10.5 mg/kg, 10.6 mg/kg, 10.7 mg/kg, 10.8 mg/kg, 10.9 mg/kg, 11 mg/kg, 11.1 mg/kg, 11.2 mg/kg, 11.3 mg/kg, 11.4 mg/kg, 11.5 mg/kg, 11.6 mg/kg, 11.7 mg/kg, 11.8 mg/kg, 11.9 mg/kg, or 12 mg/kg, preferably 8 mg/kg or 10 mg/kg, more preferably 8 mg/kg

In some embodiments, 8 mg/kg or 10 mg/kg of the antibody-drug conjugate, pharmaceutically acceptable salt, stereoisomer, or metabolite thereof, or a solvate of any of the foregoing, is administered to a patient by intravenous infusion once every three weeks.

In some embodiments, the prevention and/or treatment comprises administering to a patient a therapeutically effective amount of the anti-B7-H3 antibody-drug conjugate of formula (I), a pharmaceutically acceptable salt, stereoisomer, or metabolite thereof, or a solvate of any of the foregoing, wherein the patient is a pathologically confirmed patient with unresectable locally advanced, recurrent, or metastatic cancer or another advanced solid tumor patient, preferably a patient with unresectable locally advanced, recurrent, or metastatic cancer who has progressed after or is intolerant to at least first-line treatment.

In some embodiments, the patient is a patient with unresectable locally advanced, recurrent, or metastatic esophageal squamous cell carcinoma who has progressed after or is intolerant to at least first-line treatment; and/or a patient with unresectable locally advanced, recurrent, or metastatic esophageal adenocarcinoma or adenocarcinoma of the esophagogastric junction who has progressed after or is intolerant to at least first-line treatment.

In some embodiments, the unresectable locally advanced or recurrent esophageal squamous cell carcinoma patient who has progressed after or is intolerant to at least first-line treatment is a patient with unresectable locally advanced or locally recurrent esophageal squamous cell carcinoma who needs to have received, at the locally advanced or recurrent stage, at least (radical) concurrent chemoradiotherapy or sequential chemoradiotherapy; the metastatic esophageal squamous cell carcinoma patient who has progressed after or is intolerant to at least first-line treatment is a metastatic esophageal squamous cell carcinoma patient who has received at least platinum-containing chemotherapy.

In some embodiments, the patient with unresectable locally advanced or locally recurrent esophageal adenocarcinoma or adenocarcinoma of the esophagogastric junction is required to have received at least (radical) concurrent chemoradiotherapy or sequential chemoradiotherapy.

In some embodiments, the metastatic esophageal adenocarcinoma or adenocarcinoma of the esophagogastric junction patient is a HER-2-positive metastatic esophageal adenocarcinoma or adenocarcinoma of the esophagogastric junction patient, or a HER-2-negative metastatic esophageal adenocarcinoma or adenocarcinoma of the esophagogastric junction patient.

In some embodiments, the HER-2-positive metastatic esophageal adenocarcinoma or adenocarcinoma of the esophagogastric junction patient has received at least anti-HER-2 therapy combined with platinum-containing chemotherapy; the HER-2-negative metastatic esophageal adenocarcinoma or adenocarcinoma of the esophagogastric junction patient has received at least platinum-containing chemotherapy.

In some embodiments, the other advanced solid tumor patient is another advanced solid tumor patient for whom there is no standard treatment regimen or who has failed standard treatment.

Further, the method comprises selecting the patient based on an elevated level of a biomarker related to cancer.

In some embodiments, evaluation of the therapeutic effect comprises detecting the concentration of a biomarker in a patient sample, and preferably, the biomarker is selected from soluble B7-H3 and PD-L1.

Further, by determining the soluble B7-H3 concentration detected from patient blood samples collected during

the screening period, during treatment, and at the end-of-treatment visit and/or collected for determining the PD-L1 protein expression in patient tumor tissue samples, the relationship between the biomarker and the efficacy of the antibody-drug conjugate is evaluated.

In some embodiments, the prevention and/or treatment comprises administering to a patient a therapeutically effective amount of the anti-B7H3 antibody-drug conjugate of formula (I) or a pharmaceutically acceptable salt, stereoisomer, metabolite, or solvate compound thereof, wherein the route of administration is oral administration, parenteral administration, or transdermal administration, and the parenteral administration is selected from intravenous injection, intravenous infusion, subcutaneous injection, and intramuscular injection, preferably intravenous infusion.

In some embodiments, the prevention and/or treatment comprises administering to a patient a therapeutically effective amount of the anti-B7H3 antibody-drug conjugate of formula (I), a pharmaceutically acceptable salt, stereoisomer, or metabolite thereof, or a solvate of any of the foregoing; the medicine is in an injectable form, such as a formulation for subcutaneous injection or intravenous infusion; wherein the preferred injectable form is an injection solution or lyophilized powder for injection, which comprises the anti-B7H3 antibody-drug conjugate of formula (I) or a pharmaceutically acceptable salt, stereoisomer, metabolite, or solvate compound thereof, as well as a buffer, a stabilizer, a pH regulator, and an optionally present surfactant.

Another aspect of the present invention further provides a pharmaceutical composition, comprising the above-mentioned antibody-drug conjugate and one or more pharmaceutically acceptable excipients.

The anti-B7H3 antibody-drug conjugate provided by the present invention can significantly inhibit the growth of tumors in a human esophageal cancer subcutaneous xenograft mouse model, and the tumor-bearing mice can tolerate the combined doses well, with no significant body-weight loss observed; when used in clinical trials for treating cancer, it has improved safety, reduced drug toxicity, can effectively increase patient ORR, and has excellent clinical application prospects.

## Description of the Drawings

Figure 1 Efficacy of Drug A or B in a human esophageal squamous cell carcinoma subcutaneous xenograft mouse model.

Figure 2 Study flow chart of the phase II clinical study of the present invention.

## Detailed Description

### Terms

For easier understanding of the present disclosure, certain technical and scientific terms are specifically defined below. Unless otherwise expressly defined herein, all other technical and scientific terms used herein have the meanings commonly understood by a person of ordinary skill in the art to which the present disclosure belongs.

The present disclosure incorporates the entire contents of application WO2020063673 into the present application.

The term "antibody-drug conjugate" refers to an antibody being linked to a biologically active drug through a stable linking unit. In the present disclosure, "antibody-drug conjugate" refers to linking a monoclonal antibody or an antibody fragment to a biologically active toxic drug through a stable linking unit.

The term "antibody" refers to an immunoglobulin, which is a tetrameric structure composed of two identical heavy chains and two identical light chains linked by interchain disulfide bonds. The amino acid composition and arrangement order of the constant region of immunoglobulin heavy chains are different, and therefore their antigenicity is also different. Accordingly, immunoglobulins can be divided into five classes, also called immunoglobulin isotypes, namely IgM, IgD, IgG, IgA and IgE, and their corresponding heavy chains are respectively the $\mu$ chain, $\delta$ chain, $\gamma$ chain, $\alpha$ chain, and $\varepsilon$ chain. The same class of Ig can further be divided into different subclasses according to differences in the amino acid composition of its hinge region and the number and positions of heavy-chain disulfide bonds, for example, IgG can be divided into IgG1, IgG2, IgG3, and IgG4. Light chains are divided, according to differences in the constant region, into $\kappa$ chains or $\lambda$ chains. Among the five classes of Ig, each class of Ig can have a $\kappa$ chain or a $\lambda$ chain.

The sequences of about 110 amino acids near N terminus of the antibody heavy chains and light chains vary greatly and are variable regions (the Fv region); the remaining amino acid sequences near the C terminus are relatively stable and are constant regions. The variable region comprises 3 hypervariable regions (HVR) and 4 framework regions with relatively conserved sequences (FR). The 3 hypervariable regions determine the specificity of the antibody, and are also called complementarity-determining regions (CDR). Each light-chain variable region (LCVR) and heavy-chain variable region (HCVR) is composed of 3 CDR regions and 4 FR regions, and the order of their arrangement from the amino terminus to the carboxyl terminus is: FR1, CDR1, FR2, CDR2, FR3, CDR3, FR4. The 3 CDR regions of the light

chain refer to LCDR1, LCDR2, and LCDR3; the 3 CDR regions of the heavy chain refer to HCDR1, HCDR2, and HCDR3.

In the present disclosure, the amino acid sequences of the above CDRs are all shown according to the Kabat definition rules. However, it is well known to those skilled in the art that antibody CDRs can be defined by a variety of methods in the art, for example, Chothia based on the three-dimensional structure of antibodies and the topology of CDR loops (Chothia et al. (1989) Nature 342:877-883, Al-Lazikani et al., "Standard conformations for the canonical structures of immunoglobulins", Journal of Molecular Biology, 273, 927-948 (1997)), Kabat based on antibody sequence variability (Kabat et al., Sequences of Proteins of Immunological Interest, 4th edition, U.S. Department of Health and Human Services, National Institutes of Health (1987)), AbM (University of Bath), Contact (University College London), the international ImMunoGeneTics database (IMGT) (World Wide Web imgt.cines.fr/), and the North CDR definition based on affinity propagation clustering using a large number of crystal structures. Those skilled in the art should understand that, unless otherwise specified, the terms "CDR" and "complementarity-determining region" of a given antibody or a region thereof (for example, a variable region) should be understood as encompassing complementarity-determining regions as defined by any one of the above known solutions described in the present invention. Although the scope of protection claimed by the present invention is based on the sequences shown according to the Kabat definition rules, corresponding amino acid sequences under other CDR definition rules should also fall within the scope of protection of the present invention.

The term "antigen-binding fragment" refers to one or more fragments of an antibody that retain the ability to specifically bind an antigen. It has been shown that fragments of full-length antibodies can be used to carry out the antigen-binding function of the antibodies." Examples of binding fragments included in the "antigen-binding fragment" include (i) a Fab fragment, a monovalent fragment composed of VL, VH, CL, and CH1 domains; (ii) an $F(ab')_2$ fragment, a bivalent fragment comprising two Fab fragments linked by a disulfide bridge on the hinge region; (iii) an Fd fragment composed of VH and CH1 domains; (iv) an Fv fragment composed of the VH and VL domains of one arm of an antibody; (v) a single-domain or dAb fragment (Ward et al., (1989) Nature 341: 544-546), which is composed of a VH domain; and (vi) isolated complementarity-determining regions (CDRs) or (vii) a combination of two or more isolated CDRs optionally linked by a synthetic linker.

The term "drug load" refers to the average number of cytotoxic drugs loaded on each ligand in a formula (I) molecule, and can also be expressed as the ratio of drug amount to antibody amount; the range of drug load can be that each antibody (Pc) is linked to 0-12, preferably 1-10 cytotoxic drugs (D). In embodiments of the present disclosure, the drug load is represented as n, and may also be called the DAR value, exemplarily the mean value of 1, 2, 3, 4, 5, 6, 7, 8, 9, and 10. The average number of drugs per ADC molecule after the conjugation reaction can be characterized by conventional methods such as UV/visible spectroscopy, mass spectrometry, ELISA assay, and HPLC.

The term "treatment" means administering to a patient a therapeutic agent for internal or external administration, for example, a composition comprising any one of the binding compounds of the present disclosure, wherein the patient has one or more disease symptoms, and the therapeutic agent is known to have a therapeutic effect on these symptoms. Usually, the therapeutic agent is administered to the treated patient or population in an amount effective to alleviate one or more disease symptoms, so as to induce regression of such symptoms or inhibit the development of such symptoms to any clinically measurable extent. The amount of a therapeutic agent effective to alleviate any specific disease symptom (also referred to as a "therapeutically effective amount") can vary according to various factors, such as the disease condition, age, and body weight of the patient, as well as the ability of the drug to achieve the desired therapeutic effect in the patient. Whether a disease symptom has been alleviated can be evaluated by any clinical detection method usually used by a doctor or other healthcare professionals to evaluate the severity or progression of that symptom. Although the embodiments of the present disclosure (for example, treatment methods or articles) may not be effective in alleviating each target disease symptom, as determined according to any statistical test method known in the art such as the Student t test, chi-square test, Mann and Whitney U test, Kruskal-Wallis test (H test), Jonckheere-Terpstra test, and Wilcoxon test, they should alleviate the target disease symptoms in a statistically significant number of patients.

"Pharmaceutical composition" is a product comprising one or more active ingredients (for example, antibodies, antibody-drug conjugates, small-molecule drugs) in optionally specific amounts, as well as any product directly or indirectly produced by combining one or more active ingredients in optionally specific amounts. These compositions may also optionally comprise suitable pharmaceutical excipients, such as pharmaceutical carriers and pharmaceutical excipients known in the art, including buffers. Different active ingredients in the pharmaceutical composition may each be independently administered in separate formulation forms, including combined synergistic administration simultaneously or at different time points. In the present disclosure, "pharmaceutical composition" and "pharmaceutical formulation" are not mutually exclusive.

"Pharmaceutically acceptable salt" refers to a salt of the antibody-drug conjugate of the present invention, such salt having safety and efficacy when used in mammals, and having the due biological activity. The antibody-drug conjugate of the present invention contains at least one amino group, and therefore can form salts with acids, and non-limiting examples of pharmaceutically acceptable salts include: hydrochloride, hydrobromide, hydroiodide, sulfate, bisulfate, citrate, acetate, succinate, ascorbate, oxalate, nitrate, sorbate, hydrogen phosphate, dihydrogen phosphate, salicylate,

hydrogen citrate, tartrate, maleate, fumarate, formate, benzoate, methanesulfonate, ethanesulfonate, benzenesulfonate, and p-toluenesulfonate. "Pharmaceutically acceptable salt" and "pharmaceutically usable salt" may be used interchangeably.

"Solvate compound" refers to a pharmaceutically usable solvate compound formed by the antibody-drug conjugate compound of the present invention with one or more solvent molecules, and non-limiting examples of solvent molecules include: water, ethanol, acetonitrile, isopropanol, and ethyl acetate.

"Drug load" (DAR) is represented by y, that is, the average number of cytotoxic drugs per antibody of formula (I). The drug load range in the present invention may be 1-20 cytotoxic drugs (D) for each antibody. The antibody-drug conjugate of general formula (A) is a collection of antibodies conjugated with a certain range (1-20) of cytotoxic drugs. The drug load (DAR) in the antibody-drug conjugate resulting from the conjugation reaction can be characterized by conventional means, such as mass spectrometry, HPLC, and ELISA. By these means, the quantitative distribution of the antibody-drug conjugate at the y value can be determined.

The present application will be explained in more detail below in conjunction with the examples, and the examples of the present application are only used to illustrate the technical solutions of the present application, and are not intended to limit the substance and scope of the present application.

**Example 1. Investigational drug**

Anti-B7H3 antibody-drug conjugate for injection

Preparation of anti-B7H3 antibody-drug conjugate: according to the production method described in WO2020063673, h1702DS (anti-B7H3 antibody) and an exatecan analog were used to prepare the anti-B7H3 antibody-drug conjugate shown in the following structure, and the average value calculated by the HIC method was: n = 4.1, namely FADC-2. Of them, the h1702DS heavy-chain sequence is as shown in SEQ ID NO: 09, and the light-chain sequence is as shown in SEQ ID NO: 10.

FADC-2

Dosage form: injection (lyophilized powder)
Specification: 100 mg/vial, packaged in a 20 mL injection vial made of medium borosilicate glass tubing
Route of administration: intravenous infusion

**Example 2. Evaluation of the in vivo inhibitory effect of the anti-B7H3 antibody-drug conjugate in a human esophageal squamous cell carcinoma subcutaneous xenograft mouse model**

1. Experimental materials

Drug A: anti-B7H3 antibody-drug conjugate, physiological saline was used for drug preparation;
Drug B: anti-B7H3 antibody, physiological saline was used for drug preparation;

Human esophageal squamous cell carcinoma tumor-bearing mouse tumor tissues ES0136, ES0204, ES0218, and ES0219 were sourced from Crown Bioscience (Taicang) Inc.; BALB/c nude mice, female, 6-7 weeks old, body weight 16-23 g, were purchased from Jiangsu Jicui Yaokang Biotechnology Co., Ltd.

2. Experimental method

Tumor tissues were collected from mice bearing human esophageal squamous cell carcinoma xenografts, cut into tumor pieces with a diameter of 2-3 mm, and inoculated subcutaneously at the right anterior scapular region of BALB/c nude mice. When the average tumor volume of the tumor-bearing mice reached 150-200 $mm^3$, the mice were randomly grouped. The coefficient of variation (CV) of tumor volume within each group was calculated by the formula CV = SD/MTV × 100%, and should be less than 30%. Administration began on the day of grouping, and the day of grouping was defined as Day 0. Drug A or Drug B was administered via the tail vein (i.v.) once weekly (QW); the administration volume was 10 mL/kg; the specific administration doses and administration regimens are shown in Table 1. Tumor volume was measured, mouse body weight was weighed, and data was recorded.

The study endpoints were tumor growth-related parameters, specifically T/C% and TGI (%).

Tumor diameter was measured with a vernier caliper, and the calculation formula for tumor volume (V) was: V = 1/2 × a × b2, wherein a and b respectively represent the long diameter and short diameter of the tumor.

$$\text{Tumor growth inhibition rate (TGI) (\%)} = 100 - \text{T/C (\%)}.$$

When tumor regression occurred, tumor growth inhibition rate (TGI) (%) = 100 - (T - Ti)/Ti × 100.

If the tumor shrinks relative to the initial volume, that is, when T < Ti or C < Ci, it is defined as partial tumor regression (PR); if the tumor disappears completely, it is defined as complete tumor regression (CR).

At the end of the experiment, upon reaching the experimental endpoint, or when the average tumor volume reached 2000 $mm^3$, the mice were euthanized, followed by dissection to collect tumors and to take photographs thereof.

Experimental data were analyzed and plotted using GraphPad Prism 9.1.2. All experimental results are expressed as mean tumor volume ± SEM (standard error of the mean). For the statistical analysis among different groups, the optimal evaluation time point was selected (usually after the last administration). The independent-samples t-test method was used to compare whether there was a significant difference in the relative tumor volume of the treatment group as compared with the control group. $p < 0.05$ was defined as a statistically significant difference.

**Table 1. Dosing regimens** of Drug **A or B in human esophageal squamous cell carcinoma models**

| Model | Group | Number of animals | Dosing group | Dose (mg/kg) | Route of administration | Actual dosing schedule |
|---|---|---|---|---|---|---|
| ES0136 | 1 | 3 | B | 5 | *i.v.* | QW × 3 times |
| | 2 | 3 | A | 5 | *i.v.* | QW × 3 times |
| ES0204 | 1 | 3 | B | 3 | *i.v.* | QW × 3 times |
| | 2 | 3 | A | 3 | *i.v.* | QW × 3 times |
| ES0218 | 1 | 3 | B | 5 | *i.v.* | QW × 3 times |
| | 2 | 3 | A | 5 | *i.v.* | QW × 3 times |
| ES0219 | 1 | 3 | B | 5 | *i.v.* | QW × 3 times |
| | 2 | 3 | A | 5 | *i.v.* | QW × 3 times |

**Experimental** results

The growth inhibitory effect of Drug A or B on human esophageal squamous cell carcinoma models is shown in Table 2.

**Table 2. Growth inhibitory effect of Drug A or B on human esophageal squamous cell carcinoma models**

| Model | Group | Tumor volume[a] ($\bar{x}$ ±s) | TGI (%) | *P* value[b] (As compared with the control group) |
|---|---|---|---|---|
| ES0136 | G1, B, 5 mg/kg | 1866.55±70.02 | - | - |
| | G2, A, 5 mg/kg | 45.01±22.64 | 171.20 | < 0.001 |
| ES0204 | G1, B, 3 mg/kg | 2222.75±177.97 | - | - |
| | G2, A, 3 mg/kg | 178.91±168.02 | 98.31 | 0.001 |

(continued)

| Model | Group | Tumor volume[a] ($\bar{x}$ ±s) | TGI (%) | P value[b] (As compared with the control group) |
|---|---|---|---|---|
| ES0218 | G1, B, 5 mg/kg | 2078.92±602.01 | - | - |
| | G2, A, 5 mg/kg | 134.56±52.77 | 124.94 | 0.032 |
| ES0219 | G1, B, 5 mg/kg | 1958.45±56.67 | - | - |
| Model | Group | Tumor volume[a] ( $x$ ±S) | TGI (%) | P value[b] (As compared with the control group) |
| | G2, A, 5 mg/kg | 0±0 | 200 | < 0.001 |

Note:
1. Data are expressed as "mean value ± standard error";
2. When there is no tumor regression, TGI% = $(1-(T_i-T_0)/(C_i-C_0)) \times 100\%$; when there is tumor regression, TGI% = $(1-(T_i-T_0)/T_0) \times 100\%$;
3. The *p* value was obtained by comparing the TV of the two groups using the t-test method.

In the ES0136 model, the average tumor volume of mice in the control group on the day the experiment ended was 1866.55 mm$^3$. In the test drug **A** (5 mg/kg, QW × 3 times) treatment group, the average tumor volume was 45.01 mm$^3$ on the day the experiment ended, which was statistically significantly different as compared with that in the control group ($p < 0.001$), and the relative tumor growth inhibition rate TGI (%) was 171.20%.

In the ES0204 model, the average tumor volume of mice in the control group on the day the experiment ended was 2222.75 mm$^3$. In the test drug A (3 mg/kg, QW × 3 times) treatment group, the average tumor volume was 178.91 mm$^3$ on the day the experiment ended, which was statistically significantly different as compared with that in the control group ($p = 0.001$), and the relative tumor growth inhibition rate TGI (%) was 98.31%.

In the ES0218 model, the average tumor volume of mice in the control group on the day the experiment ended was 2078.92 mm$^3$. In the test drug A (5 mg/kg, QW × 3 times) treatment group, the average tumor volume was 134.56 mm$^3$ on the day the experiment ended, which was statistically significantly different as compared with that in the control group ($p = 0.032$), and the relative tumor growth inhibition rate TGI (%) was 124.94%.

In the ES0219 model, the average tumor volume of mice in the control group on the day the experiment ended was 1958.45 mm$^3$. In the test drug A (5 mg/kg, QW × 3 times) treatment group, the average tumor volume was 0.00 mm$^3$ on the day the experiment ended, which was statistically significantly different as compared with that in the control group ($p < 0.001$), and the relative tumor growth inhibition rate TGI (%) was 200%.

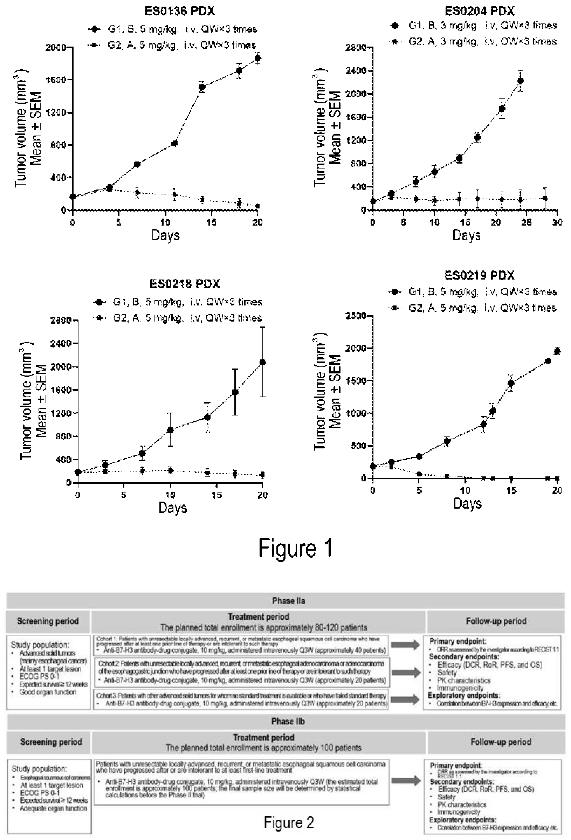

Throughout the experiment, the tumor-bearing mice tolerated the tested dose levels well, and no obvious symptom of body weight reduction occurred. The in vivo efficacy of Drug A and Drug B in the human esophageal cancer model is shown in Figure 1.

**4. Experimental conclusion:**

In the human esophageal squamous cell carcinoma subcutaneous xenograft mouse model, Drug A can significantly inhibit tumor growth.

**Example 3. Clinical study of the anti-B7H3 antibody-drug conjugate for injection in solid tumors**

I. Phase I clinical study of the safety, tolerability, pharmacokinetics, and efficacy of the anti-B7H3 antibody-drug conjugate for injection in patients with advanced solid tumors

1. Study objectives

Phase Ia (dose-escalation period) To evaluate the safety and tolerability of intravenous administration of the anti-B7H3 antibody-drug conjugate for injection in patients with advanced solid tumors. To evaluate the PK characteristics of intravenous administration of the anti-B7H3 antibody-drug conjugate for injection in patients with advanced solid tumors; to evaluate the efficacy of intravenous administration of the anti-B7H3 antibody-drug conjugate for injection in treating advanced solid tumors; to evaluate the immunogenicity of intravenous administration of the anti-B7H3 antibody-drug conjugate for injection in patients with advanced solid tumors; to evaluate the relationship between B7-H3 protein expression in tumor tissues and efficacy. Phase Ib (dose-expansion period) To evaluate the efficacy of intravenous administration of the anti-B7H3 antibody-drug conjugate for injection in patients with advanced solid tumors. To evaluate the safety and tolerability of intravenous administration of the anti-B7H3 antibody-drug conjugate for injection in patients

with advanced solid tumors; to evaluate the PK characteristics of intravenous administration of the anti-B7H3 antibody-drug conjugate for injection in patients with advanced solid tumors; to evaluate the immunogenicity of intravenous administration of the anti-B7H3 antibody-drug conjugate for injection in patients with advanced solid tumors; to evaluate the relationship between B7-H3 protein expression in tumor tissues and efficacy.

2. Subjects

1) Inclusion criteria

1. Male or female aged 18 years or older (≥ 18 years old)
2. Patients with pathologically confirmed advanced solid tumors who have failed standard treatment or are intolerant to standard treatment
3. According to RECIST 1.1, the subject has at least 1 target lesion.
4. Fresh or archived tumor tissue samples are required.
5. ECOG PS score is 0-1 and has not worsened within 2 weeks before the first administration.
6. Minimum expected survival is greater than 12 weeks.
7. Female subjects of childbearing potential shall be willing to use appropriate contraceptive measures and should not breastfeed from the time of signing informed consent until 6 months after treatment discontinuation; male subjects shall be willing to use barrier contraception (that is, condoms) from signing the informed consent to 6 months after termination of treatment.
8. For female subjects, within 7 days before the first administration, the blood pregnancy test result is negative, or they meet the requirements to demonstrate that there is no risk of pregnancy.
9. Voluntarily participate in this clinical trial, understand the study procedures, and be able to sign the informed consent form in writing.

2) Exclusion criteria

1. If a subject meets any one of the following criteria, the subject cannot be enrolled in this study: Having received or currently undergoing the following treatments: a) Has received or is currently receiving treatment targeting B7-H3. b) Within 14 days before the first administration of study treatment, has received cytotoxic chemotherapeutic drugs, investigational drugs, traditional Chinese medicine treatment with an antitumor indication, or other antitumor drugs; or needs to continue receiving these drug treatments during the study. c) Has received macromolecular antitumor drug treatment within 28 days before the first administration of study treatment. d) Has received local radiotherapy within 2 weeks before the first administration of study treatment; within 4 weeks before the first administration of study treatment, has received irradiation to more than 30% of the bone marrow, or has received large-area radiotherapy. e) Has pleural effusion/ascites requiring clinical intervention; has pericardial effusion. f) Within 4 weeks before the first administration of study treatment, has undergone major surgery. g) Spinal cord compression or brain metastasis; patients who, during or after the last treatment before screening, has had disease progression due to brain metastasis and has not be treated and has meningeal metastasis or brainstem metastasis; patients with spinal cord compression. h) Within 7 days before the first administration of the study drug, has used strong inhibitors or strong inducers of CYP3A4, CYP2D6, P-gp, or BCRP, or drugs that are sensitive substrates of CYP3A4, CYP2D6, P-gp, or BCRP with a narrow therapeutic window; or needs to continue receiving these drug treatments during the study. i) Is receiving drug treatment known to prolong the QT interval or that may cause torsades de pointes ventricular tachycardia; or needs to continue receiving these drug treatments during the study.
2. Has toxicity remaining from prior treatment of grade ≥ 2 according to the Common Terminology Criteria for Adverse Events (CTCAE version 5.0) (except alopecia and residual neurotoxicity).
3. Has a history of other primary solid tumors
4. Has insufficient bone marrow reserve or hepatic or renal organ function, meeting any one of the following laboratory limits: a) Neutrophil count < 1.5×10^9/L b) Platelet count < 90×10^9/L c) Hemoglobin < 90 g/L d) Total bilirubin > 1.5× upper limit of normal (ULN); if there is confirmed Gilbert syndrome or liver metastasis, total bilirubin > 3.0×ULN e) ALT and/or AST > 2.5×ULN; if liver metastasis is present, then ALT and/or AST > 5.0×ULN f) Creatinine > 1.5×ULN and creatinine clearance < 50 mL/min; creatinine clearance needs to be confirmed only when creatinine > 1.5×ULN; g) International normalized ratio > 1.5, and activated partial thromboplastin time APTT > 1.5×ULN h) Serum albumin < 28 g/L.

5. Meeting any one of the following cardiac examination criteria: a) Fridericia-corrected QT interval (average QTcF value > 470 msec) obtained from electrocardiogram (ECG) examination at rest; b) Resting ECG suggests any clinically significant rhythm, conduction, or ECG morphological abnormality judged important by the investigator; c) Presence of any factor increasing the risk of QTc prolongation or arrhythmic events, such as heart failure, refractory hypokalemia, congenital long QT syndrome, family history of long QT syndrome, unexplained sudden death of a first-degree relative under 40 years old, or any concomitant drug that prolongs the QT interval; d) Left ventricular ejection fraction < 50%.

6. Has severe, uncontrolled, or active cardiovascular disease.

7. Patients who have had diabetic ketoacidosis or hyperglycemic hyperosmolar state within 6 months before the first administration and whose glycated hemoglobin test value during the screening period is ≥ 7.5%

8. Has severe or poorly controlled hypertension.

9. Has had clinically significant bleeding symptoms or an obvious bleeding tendency within 1 month before the first administration

10. Has had a severe arterial or venous thrombotic event within 3 months before the first administration

11. Has had a severe infection within 4 weeks before the first administration; active infection having received therapeutic intravenous antibiotics within 2 weeks before the first administration or oral antibiotics within 1 week before the first administration. Patients currently receiving or having received prophylactic antibiotic treatment may be enrolled.

12. Having received continuous glucocorticoid treatment for more than 30 days within 30 days before the first administration, or needing long-term (≥ 30 days) glucocorticoid treatment, or having other acquired or congenital immunodeficiency diseases, or having a history of organ transplantation.

13. Known to have an active infectious disease, such as active hepatitis B or hepatitis C, tuberculosis, syphilis, or human immunodeficiency virus HIV infection, etc. Active infectious diseases will not be proactively screened.

14. Currently suffering from hepatic encephalopathy, hepatorenal syndrome, or liver cirrhosis of ≥ Child-Pugh class B.

15. Other moderate to severe pulmonary diseases that may interfere with the detection or management of drug-related pulmonary toxicity and seriously affect respiratory function

16. A previous history of severe neurological or psychiatric disorders.

17. Female patients who are pregnant, breastfeeding, or planning to become pregnant during the study period.

18. Having been vaccinated or having had any degree of allergic or hypersensitivity reaction within 4 weeks before the first administration.

19. Those who have a previous history of severe allergy, or who have had a severe infusion reaction, or who are allergic to recombinant human- or murine-derived protein substances.

20. Allergic to any component of the anti-B7H3 antibody-drug conjugate for injection.

21. Patients who, in the investigator's judgment, may have poor compliance with the procedures and requirements of the study.

22. Patients who, in the investigator's judgment, have any condition that endangers patient safety or interferes with study assessment.

3. Dosing regimen

Administration by intravenous infusion; the total dose to be administered of the anti-B7H3 antibody-drug conjugate for injection is calculated according to the body weight measurement result of the subject before each administration. The dose-escalation stage will begin from the lowest preset dose of 1.0 mg/kg, and will be escalated sequentially according to the preset dose groups of 1.0 mg/kg, 2.0 mg/kg, 4.0 mg/kg, 6.0 mg/kg, and 8.0 mg/kg

Medication schedule: once every 3 weeks (Q3W, 21 days as one treatment cycle)

4. Study endpoints

1) Primary study endpoints

| Serial number | Indicator | Evaluation time | Endpoint indicator selection |
|---|---|---|---|
| 1 | Phase Ia (dose-escalation period): MTD or MAD of intravenous injection of the anti-B7H3 antibody-drug conjugate for injection; | Within 21 days after a single administration | Safety indicator |
| 2 | Phase Ib (dose-expansion period): ORR evaluated by the investigator according to the RECIST 1.1 standard. | Tumor imaging assessment is performed once every 6 weeks after C1D1, and once every 12 weeks after 24 weeks, until the subject has disease progression or withdraws from the study. | Efficacy indicator |

2) Secondary study endpoints

| Serial number | Indicator | Evaluation time | Endpoint indicator selection |
|---|---|---|---|
| 1 | Phase Ia and Phase Ib: safety of intravenous injection of the anti-B7H3 antibody-drug conjugate for injection | Entire study period | Safety indicator |
| 2 | Phase Ia and Phase Ib: pharmacokinetic characteristics of intravenous injection of the anti-B7H3 antibody-drug conjugate for injection. | Entire study period | Safety indicator |
| 3 | Phase Ia and Phase Ib: efficacy of intravenous injection of the anti-B7H3 antibody-drug conjugate for injection: objective response rate (ORR), disease control rate (DCR), duration of response (DoR), and progression-free survival (PFS) as evaluated according to the RECIST 1.1 criteria. | Tumor imaging assessment is performed once every 6 weeks after C1D1, and once every 12 weeks after 24 weeks, until the subject has disease progression or withdraws from the study. | Efficacy indicator |
| 4 | Phase Ia and Phase Ib: immunogenicity of intravenous injection of the anti-B7H3 antibody-drug conjugate for injection: antibodies against the anti-B7H3 antibody-drug conjugate for injection (ADA). | Entire study period | Safety indicator |
| 5 | Phase Ia and Phase Ib: correlation between B7-H3 protein expression levels in different tumor tissues and efficacy. | At study completion | Efficacy indicator |
| 6 | Phase Ib: overall survival (OS). | Entire study period | Efficacy indicator |

**II. Phase II clinical study of the anti-B7H3 antibody-drug conjugate in patients with advanced esophageal cancer and other advanced solid tumors**

**Overall study design**

This is a multicenter, open-label, single-arm, non-randomized Phase II clinical study, evaluating the efficacy, safety, pharmacokinetic characteristics, and immunogenicity of intravenous administration of the anti-B7H3 antibody-drug conjugate in patients with unresectable locally advanced, recurrent, or metastatic esophageal cancer or other advanced solid tumors.

The phase IIa clinical study will evaluate the efficacy, safety, PK characteristics, and immunogenicity of the anti-B7H3 antibody-drug conjugate in subjects with unresectable locally advanced, recurrent, or metastatic esophageal cancer or adenocarcinoma of the esophagogastric junction who have progressed after or are intolerant to at least first-line treatment, as well as in other patients with advanced solid tumors for whom no standard treatment is available or whose

standard treatment has failed.

The phase IIb clinical study will evaluate the efficacy, safety, PK characteristics, and immunogenicity of the anti-B7H3 antibody-drug conjugate in subjects with unresectable locally advanced, recurrent, or metastatic esophageal squamous cell carcinoma who have progressed after or are intolerant to at least first-line treatment. According to the results of the phase IIa study, the study population of the phase IIb study may be adjusted.

The study flow chart is as shown in Figure 2.

**1. Study stage:**

**Phase IIa**

At this stage, expansion studies will be carried out in the following three populations:

Cohort 1: patients with unresectable locally advanced, recurrent, or metastatic esophageal squamous cell carcinoma who have progressed after or are intolerant to at least first-line treatment;
Cohort 2: patients with unresectable locally advanced, recurrent, or metastatic esophageal adenocarcinoma or adenocarcinoma of the esophagogastric junction who have progressed after or are intolerant to at least first-line treatment;
Cohort 3: other advanced solid tumor patients for whom there is no standard treatment regimen or who have failed standard treatment

At this stage, early proof-of-concept studies will be conducted in Cohorts 1, 2, and 3 at dose levels of 8.0 mg/kg or 10.0 mg/kg. At this study stage, it is expected that about 80-120 subjects will be enrolled (Cohort 1: 40 cases, Cohort 2: about 20 cases, Cohort 3: about 20 cases). Based on the accumulation of phase IIa trial data, translational medicine research data, and research progress of similar products, the expansion-stage study population may be adjusted. If a new expansion cohort is added, that cohort will recruit up to about 40 subjects.

**Dosing regimen**: all subjects in this study will continuously receive the anti-B7H3 antibody-drug conjugate by intravenous infusion, once every 3 weeks (Q3W), with 21 days as one treatment cycle, and dosing will continue until objective disease progression (excluding any drug give-away) or until other criteria for termination of study treatment specified in the protocol are met.

**Efficacy assessment**: within 28 days before the first administration (C1D1), all subjects are required to undergo baseline tumor assessment, such as contrast-enhanced CT of the neck, contrast-enhanced CT of the chest, contrast-enhanced CT of the abdomen (including the pelvis), contrast-enhanced MRI of the head (preferred)/contrast-enhanced CT, bone scan, and imaging examinations of other sites where metastasis is suspected. According to the Response Evaluation Criteria in Solid Tumors (RECIST v1.1), subjects will undergo tumor imaging assessment once every 6 weeks within 24 weeks after C1D1, and once every 12 weeks starting from Week 25, until the subject has disease progression or withdraws from the study.

**Safety assessment**: within 28 days before the first administration (C1D1), all subjects are required to undergo screening safety assessments, and after being assessed as meeting the enrollment criteria, are required to undergo baseline safety examinations before the first administration. After all subjects are enrolled in the study, safety assessments will be performed in each treatment cycle, and the assessments include physical examination, vital signs, laboratory examinations, electrocardiogram, etc., (see Appendix II for details), until 30 days after the last administration. All subjects will continue to undergo safety follow-up after the end of treatment, until 90 days after the last administration.

**Survival follow-up**: starting from the time when the investigator determines termination of treatment, survival follow-up is conducted according to the process of once every 4 cycles (12 weeks).

**PK and immunogenicity study**: all subjects will undergo PK and immunogenicity blood sample collection after the first administration and during continuous administration, to evaluate the PK characteristics and immunogenicity of the anti-B7H3 antibody-drug conjugate.

**Exploratory studies**:

- Study of the relationship between B7-H3 protein expression in baseline tumor tissues and the efficacy of the anti-B7H3 antibody-drug conjugate: the study will collect or obtain tumor tissue samples from subjects during the screening period for B7-H3 biomarker testing, so as to evaluate the relationship between the B7-H3 protein expression level and the efficacy of the anti-B7H3 antibody-drug conjugate.
- Study of the relationship between baseline gene abnormal status and the efficacy of the anti-B7H3 antibody-drug

conjugate: the study will collect blood samples from subjects during the screening period for genetic testing, so as to evaluate the relationship between the baseline gene abnormal status and the efficacy of the anti-B7H3 antibody-drug conjugate.

- Study of the relationship between baseline PD-L1 protein expression and the efficacy of the anti-B7H3 antibody-drug conjugate: the study will collect or obtain tumor tissue samples from subjects during the screening period for PD-L1 biomarker testing, so as to evaluate the relationship between the PD-L1 protein expression and the efficacy of the anti-B7H3 antibody-drug conjugate.
- Study of the correlation between baseline sB7-H3 concentration level and baseline tumor B7-H3 protein expression: the study will collect or obtain tissue samples and blood samples from subjects during the screening period, respectively conduct testing of B7-H3 protein expression in tumor tissues and concentration testing of sB7-H3 in blood samples, so as to evaluate the correlation between the baseline sB7-H3 concentration level and the expression level of B7-H3 in tumor tissues
- Study of the relationship between sB7-H3 concentration level and the efficacy of the anti-B7H3 antibody-drug conjugate: the study will collect blood samples from subjects during the screening period, during treatment, and at the end-of-treatment visit to measure sB7-H3 levels, so as to evaluate the relationship between the baseline concentration level of sB7-H3 and the change in the concentration level of sB7-H3 during treatment and the efficacy of the anti-B7H3 antibody-drug conjugate.
- The relationship between exposure and clinical response (efficacy and safety).

**Phase IIb**

The phase IIb study will be conducted in subjects with unresectable locally advanced, recurrent, or metastatic esophageal squamous cell carcinoma who have progressed after or are intolerant to at least first-line treatment.

Dosing regimen: all subjects in phase IIb will receive study treatment of the 10.0 mg/kg dose group. At this stage, it is expected that about 100 subjects will be enrolled, and the specific number of cases will be determined by statistical calculation before the phase IIb study.

Efficacy assessment, safety assessment, exploratory studies, and survival follow-up are all the same as those in the design of the phase IIa study.

PK and immunogenicity study: all subjects will undergo PK and immunogenicity blood sample collection after the first administration and during continuous administration, to evaluate the PK characteristics and immunogenicity of the anti-B7H3 antibody-drug conjugate.

**2. Sample size determination**

The sample size of the phase IIa study is determined based on clinical considerations rather than statistical considerations, and the phase IIb sample size will be comprehensively determined according to statistical considerations and regulatory requirements. The sample size for this study must be able to provide sufficient data for safety and efficacy assessments that meet the study objectives of each phase, while exposing subjects to the study product and procedures as little as possible.

About 180-220 subjects will be enrolled in this study, and the final number of enrolled cases may be changed according to actual circumstances.

Phase IIa:

It is planned to enroll about 80-120 subjects:

Cohort 1: about 40 cases
Cohort 2: about 20 cases
Cohort 3: about 20 cases

Based on the accumulation of phase IIa trial data, translational medicine research data, and research progress of similar products, the expansion-stage study population may be adjusted. If a new expansion cohort is added, that cohort will recruit up to about 40 subjects.

Phase IIb:

The estimated total number of cases is about 100, and the specific number of cases is to be determined by statistical calculation before the phase IIb study.

**3. Randomization**

This study is a multicenter, open-label clinical study and does not involve randomization.

**4. Blinding**

This is an open-label study and does not involve blinding.

**5. Dosing regimen**

All subjects in this study will continuously receive the anti-B7H3 antibody-drug conjugate by intravenous infusion at a dosing dose of 8.0 mg/kg or 10.0 mg/kg, and the total dose of the anti-B7H3 antibody-drug conjugate is calculated according to the body weight measurement result of the subject before each administration, and the duration of the first intravenous infusion is at least 90 min. Each subsequent infusion may be adjusted according to the tolerability of the subject, but should not be less than 30 min (including the flushing stage). Once every 3 weeks (Q3W), with 21 days as one treatment cycle, until objective disease progression (excluding any drug give-away) or until other criteria for termination of study treatment specified in the protocol are met.

Note: the diluted drug solution of this product may be stored for 24 hours under conditions of 2°C-8°C or for 6 hours at room temperature (16°C-30°C). It is recommended that the intravenous infusion time be controlled within 4 hours, with a maximum of not more than 6 hours.

During the study period, if the administration time of the anti-B7H3 antibody-drug conjugate exceeds the preset dosing window period, the subsequent dosing time will be calculated from the actual date of the previous administration.

Based on the obtained study data, the dosing dose may be adjusted or another dosing regimen may be used.

**6. Phase IIa study design**

6.1. Dose selection for phase IIa

According to the comprehensive evaluation of the safety and efficacy study data from the dose-escalation stage of the anti-B7H3 antibody-drug conjugate (as of February 3, 2023), expansion studies will be conducted at the dose of 10.0 mg/kg Q3W, and the basis for dose determination is as follows:

1) In terms of safety: 16.0 mg/kg Q3W is an intolerable dose, and 12.0 mg/kg Q3W is the MTD. However, based on the accumulated longer-term safety data evaluation, in the 12.0 mg/kg dose group, the incidence of adverse events of grade ≥ 3 was 83.3% (15/18, the vast majority of which were able to recover from the adverse events), significantly higher than in the other dose groups; the incidence of adverse events leading to dose reduction was 38.9% (7/18), and 1 subject had an adverse event leading to death, indicating that the overall safety tolerability of the 12.0 mg/kg dose group was poor.

2) PK characteristics and PK/PD simulation: the PK characteristics of this product show that the exposure to the anti-B7H3 antibody-drug conjugate increases as dose increases; in combination with the mechanism of action of this product, on the premise of ensuring the safety of subjects as much as possible, it is expected that the use of a higher dose may bring better efficacy benefit. The results of model simulation show that 10.0 mg/kg is a safer and more tolerable dose than 12.0 mg/kg.

3) In terms of efficacy: antitumor treatment responses were observed at 4.0-12.0 mg/kg Q3W, and at the same time an overall trend of better efficacy as the dose increased was shown, among which relatively clear antitumor treatment effects were observed at both doses of 8.0 mg/kg Q3W and 12.0 mg/kg Q3W.

In summary, phase IIa will conduct expansion studies in advanced esophageal cancer and other advanced solid tumors at the dose of 10.0 mg/kg Q3W.

Based on the accumulation of phase IIa trial data, translational medicine research data, and research progress of similar products, the expansion-stage study population may be adjusted. If a new expansion cohort is added, that cohort will recruit up to about 40 subjects.

6.2. Setting of the phase IIa population

At this stage, expansion studies will be conducted in the following indication populations, including:

Cohort 1: patients with unresectable locally advanced, recurrent, or metastatic esophageal squamous cell carcinoma

who have progressed after or are intolerant to at least first-line treatment

Cohort 2: patients with unresectable locally advanced, recurrent, or metastatic esophageal adenocarcinoma or adenocarcinoma of the esophagogastric junction who have progressed after or are intolerant to at least first-line treatment

Cohort 3: other advanced solid tumor patients for whom there is no standard treatment regimen or who have failed standard treatment

According to the characteristics of different indications and clinical study data, the efficacy of the anti-B7H3 antibody-drug conjugate in the target population will be evaluated, and at the same time the safety, PK characteristics, and immunogenicity of intravenous administration of the anti-B7H3 antibody-drug conjugate will continue to be evaluated.

Based on the accumulation of clinical trial data, translational medicine research data, and progress in field research and development, it is possible to adjust the above cohorts.

6.3. Phase IIa grouping and enrollment rules

All subjects who meet the inclusion criteria and do not meet the exclusion criteria will be assigned enrollment numbers according to the chronological order of completion of screening, and study drug will be dispensed.

**7. Phase IIb study design**

7.1. Dose selection for phase IIb

According to the safety, PK, and efficacy data already obtained from the dose escalation of the anti-B7H3 antibody-drug conjugate study, all subjects will receive study treatment at a dose of 8.0 mg/kg or 10.0 mg/kg.

3.7.2. Setting of the phase IIb population

Phase IIb is planned to be carried out in patients with unresectable locally advanced, recurrent, or metastatic esophageal squamous cell carcinoma who have progressed after or are intolerant to at least first-line treatment.

3.7.3. Phase IIb grouping and enrollment rules

All subjects who meet the inclusion and exclusion criteria will be assigned enrollment numbers according to the chronological order of completion of screening, and study drug will be dispensed.

**8. Pharmacokinetic design**

PK sample collection: all subjects are required to undergo PK testing. During the study, the subsequent PK sample collection times may be adjusted according to the stage PK results.

Blood drug concentration determination: this study will use validated bioanalytical methods to determine the blood drug concentrations of three components of the anti-B7H3 antibody-drug conjugate (including toxin-conjugated antibody, total antibody, and free toxin). The bioanalytical laboratory will determine the blood drug concentrations of the three components in all received samples. For specific sample collection, see the central laboratory operation manual, and the detection methods will be reflected in the bioanalytical method validation report.

Pharmacokinetic analysis: according to the blood drug concentration data from multiple determinations, the pharmacokinetic parameters of the anti-B7H3 antibody-drug conjugate in the human body will be obtained; the nonlinear mixed-effects kinetic method will be used to conduct population pharmacokinetic (PopPK) analysis, and the PopPK analysis results will be reported separately.

**9. Immunogenicity design**

All subjects will undergo immunogenicity blood sample collection. The bioanalytical laboratory will conduct detection of antibodies against the anti-B7H3 antibody-drug conjugate (ADA) in all received samples, and when necessary, will determine neutralizing antibody (Nab) activity.

**10. sB7-H3 biomarker design**

All subjects will undergo blood sample collection for the sB7-H3 biomarker study.

**11. Toxicity management plan**

Toxicity in this study refers to adverse events related to the study drug, which includes events that are definitely related to and possibly related to the study drug, and unable to be determined events, and does not include events that are possibly unrelated to and definitely unrelated to the study drug.
Dose adjustment is divided into interrupted administration, delayed administration, dose reduction, and permanent discontinuation.
Interrupted administration: during infusion of the anti-B7H3 antibody-drug conjugate, if for any reason (for example, an infusion reaction) the investigator judges that the administration needs to be interrupted, then after the event is relieved or improves to Grade 1, infusion treatment may be continued at the original infusion rate or at a reduced infusion rate, and it is also possible to stop the administration for this treatment. Subjects with interrupted administration should still undergo the subsequent study procedures according to the original plan, including collection of PK samples.
Delayed administration: the planned administration time of the anti-B7H3 antibody-drug conjugate is delayed due to an adverse event. The delayed administration time of the anti-B7H3 antibody-drug conjugate must not exceed 21 days (calculated from the planned dosing time), in order to ensure the drug intensity of the treatment received by the subject. If the delayed administration time exceeds the above standard, then the study administration will be ended for the subject, unless after discussion between the investigator and the sponsor there is unanimous agreement to continue the drug administration (for example, noninfectious pneumonia requiring 28 days of recovery, delay of the drug for more than 21 days due to causes of toxic side effects not related to the drug and no disease progression having occurred, etc.).
Dose reduction: the dose permitted for reduction of the anti-B7H3 antibody-drug conjugate is a lower dose whose safety has already been verified in the dose-escalation stage. If the subject still is intolerant after reduction to 4.0 mg/kg, administration will be terminated, and no further reduction to a lower dose will be accepted, unless after communication between the investigator and the sponsor it is unanimously considered necessary to further reduce the dose. If the subject is still intolerant after reduction to the preset lowest dose, the subsequent treatment strategy will be decided through discussion by the investigator and the sponsor.
Permanent discontinuation: subjects who are determined to require permanent discontinuation according to the dose adjustment principles are not allowed to receive administration of the anti-B7H3 antibody-drug conjugate at any dose again.
If adverse events of Grade 3 and above that are unrelated to the anti-B7H3 antibody-drug conjugate occur, or Grade 2 laboratory abnormal values do not cause subject symptom/sign discomfort, or nonfatal adverse events (alopecia, decreased appetite, etc.) occur, the investigator may, at discretion, consider whether to delay or resume administration of the anti-B7H3 antibody-drug conjugate.

**Subject management**

1) Inclusion criteria

Subjects must meet the following inclusion criteria before they may be enrolled in this study:

1. Male or female aged 18 years or older (≥ 18 years old).
2. Tumor diagnosis and prior antitumor treatment history:

Pathologically confirmed unresectable locally advanced, recurrent, or metastatic esophageal cancer or other advanced solid tumors (it is necessary to
provide the key immunohistochemistry/tumor cell phenotype results required for a definitive diagnosis). The specific requirements are as follows:

Phase IIa:

Cohort 1: patients with unresectable locally advanced, recurrent, or metastatic esophageal squamous cell carcinoma who have progressed after or are intolerant to at least first-line treatment, and need to simultaneously meet the following conditions:
Requirements for prior treatment:

- Patients with unresectable locally advanced or locally recurrent esophageal squamous cell carcinoma need to have received at least (radical) concurrent chemoradiotherapy or sequential chemoradiotherapy at the locally advanced or recurrent stage, unless the patient has contraindications to radiotherapy/chemotherapy;

- Patients with metastatic esophageal squamous cell carcinoma need to have received at least platinum-containing chemotherapy

Cohort 2: patients with unresectable locally advanced, recurrent, or metastatic esophageal adenocarcinoma or adenocarcinoma of the esophagogastric junction who have progressed after or are intolerant to at least first-line treatment, and need to simultaneously meet the following conditions:
Requirements for prior treatment:

- Patients with unresectable locally advanced or locally recurrent esophageal adenocarcinoma or adenocarcinoma of the esophagogastric junction need to have received at least (radical) concurrent chemoradiotherapy or sequential chemoradiotherapy, unless the patient has contraindications to radiotherapy/chemotherapy;
- HER-2-positive metastatic esophageal adenocarcinoma or adenocarcinoma of the esophagogastric junction needs to have received at least anti-HER-2 treatment combined with platinum-containing chemotherapy;
- HER-2-negative metastatic esophageal adenocarcinoma or adenocarcinoma of the esophagogastric junction needs to have received at least platinum-containing chemotherapy.

Cohort 3: other advanced solid tumor patients, other than Cohort 1 and Cohort 2, for whom there is no standard treatment regimen or who have failed standard treatment.

Phase IIb: same population inclusion criteria as those for Phase IIa - Cohort 1.
Note: for subjects enrolled due to intolerance to the last treatment, the investigator needs to record the reason for or explanation of intolerance to the current treatment.

3. According to RECIST 1.1, the subject has at least 1 target lesion. The requirements for target lesions are: measurable lesions that have not undergone local treatment such as irradiation, or that have clearly progressed after local treatment, with a longest diameter at baseline of ≥ 10 mm (if a lymph node, the maximum short diameter is required to be ≥ 15 mm). Hollow-cavity sites such as the esophagus cannot be recorded as target lesions; having only brain lesions as target lesions is not accepted.
4. Provision of tumor tissue/blood samples:

- Fresh tumor tissue samples need to be provided (sample types are formalin-fixed, paraffin-embedded [FFPE] tumor tissue blocks or FFPE sections), for retrospective detection of B7-H3 and PD-L1 expression by the central laboratory using the IHC method; if fresh samples are not accessible, FFPE sections newly prepared from FFPE tumor tissue blocks within 2 years are acceptable;
- At the same time, peripheral blood samples need to be provided for detection of sB7-H3 levels and tumor-related gene abnormal status.

5. ECOG PS score is 0-1, and has not worsened within 2 weeks before the first administration.
6. Minimum expected survival is greater than 12 weeks.
7. Female subjects of childbearing potential shall be willing to take appropriate contraceptive measures (see Appendix XIV) and should not breastfeed from signing the informed consent to 6 months after the last administration; male subjects shall be willing to use barrier contraception (that is, condoms) from signing the informed consent to 6 months after the last administration.
8. Female subjects, within 7 days before the first administration, have a negative blood pregnancy test result, or satisfy one of the following criteria to prove that there is no risk of pregnancy:

a) Postmenopause is defined as age ≥ 60 years and amenorrhea for at least 12 months after stopping all exogenous hormone replacement therapy;
b) For women younger than 60 years old, if menstruation has stopped for 12 months or more after stopping all exogenous hormone treatment, and luteinizing hormone (LH) and follicle-stimulating hormone (FSH) levels are within the laboratory postmenopausal reference range, they may also be considered postmenopausal;
c) Having previously undergone irreversible sterilization surgery, including hysterectomy, bilateral oophorectomy, or bilateral salpingectomy, but excluding bilateral tubal ligation.

9. Voluntarily participate in this clinical study, understand the study procedures, and be able to sign the informed consent form in writing.

2) Exclusion criteria

If a subject meets any one of the following criteria, the subject cannot be enrolled in this study:

1. Having received or currently undergoing the following treatments (if, among the following criteria, a drug simultaneously meets multiple criteria, the longer period shall apply):

a) Has previously received or is currently receiving treatment targeting B7-H3, for example: MGC018, DS-7300a, ABBV-155, BAT8009, Enoblituzumab, and Omburtamab, etc.
b) Within 14 days before the first administration of study treatment, has received cytotoxic chemotherapeutic drugs, investigational drugs, traditional Chinese medicine treatment with an antitumor indication (for the list of drugs, see Appendix IV), or other antitumor drugs (including endocrine therapy, molecular targeted therapy, or biologic therapy, etc.); or needs to continue receiving these drug treatments during the study period.
c) Has received macromolecular antitumor drug treatment within 28 days before the first administration of study treatment (including immunotherapy, such as monoclonal antibody drugs and bispecific antibody drugs, etc.); or needs to continue receiving these drug treatments during the study period.
d) Has received local radiotherapy within 2 weeks before the first administration of study treatment; within 4 weeks before the first administration of study treatment, has received irradiation to more than 30% of the bone marrow (see Appendix VI for details), or has received large-area radiotherapy.
e) Has pleural effusion/ascites requiring clinical intervention (patients who do not require drainage of effusion or who have been stable for more than 1 week after drainage of effusion may be enrolled); has pericardial effusion (a small asymptomatic pericardial effusion that, in the investigator's assessment, does not require clinical intervention is allowed for enrollment). If antitumor drugs have been locally used during drainage (such as thoracic perfusion), enrollment is allowed only if a washout of at least 5 drug half-lives or 21 days (whichever is shorter) before the first administration of study treatment is also satisfied.
f) Within 4 weeks before the first administration of study treatment, has undergone major surgery (craniotomy, thoracotomy, or laparotomy). The definition of major surgical operations refers to Grade 3 and Grade 4 operations as specified in the "Administrative Measures for the Clinical Application of Medical Technology" in Appendix VIII.
g) Patients with brain metastasis (unless asymptomatic, stable before the first administration, not requiring steroid treatment for at least 2 weeks before the first administration, and with no imaging manifestation of obvious edema around the tumor lesion); patients in whom the most recently identified progression of brain metastatic disease has not undergone local or systemic antitumor treatment and for whom the interval to the first study administration is less than 4 weeks; patients with meningeal metastasis or brainstem metastasis; patients with spinal cord compression (identified by radiologic imaging, regardless of whether symptoms are present).
h) Within 7 days before the first administration of the study drug, has used strong inhibitors or strong inducers of CYP3A4, CYP2D6, P-gp, or BCRP, or drugs with a narrow therapeutic window that are sensitive substrates of CYP3A4, CYP2D6, P-gp, or BCRP; or needs to continue receiving these drug treatments during the study period (for the list of drugs, see Appendix IV).
i) Is currently receiving drug treatment that is known to prolong the QT interval or that may cause torsades de pointes ventricular tachycardia; or needs to continue receiving these drug treatments during the study period (for the list of drugs and washout times, see Appendix IV).

2. Has toxicity of grade ≥ 2 remaining from prior treatment according to the Common Terminology Criteria for Adverse Events (CTCAE version 5.0) (except alopecia and residual neurotoxicity).
3. Has a history of other primary solid tumors, except for:

a) a definitively treated solid tumor, with no activity for ≥ 5 years before enrollment in the study and with an extremely low risk of recurrence;
b) Non-melanoma skin cancer or malignant lentiginous nevus that has been adequately treated and has no evidence of disease recurrence;
c) Carcinoma in situ that has been adequately treated and has no evidence of disease recurrence, such as cervical carcinoma in situ;
d) Non-metastatic prostate cancer that has definitive treatment.

4. The tumor of a subject with esophageal cancer or esophagogastric junction cancer has any of the following characteristics: ① the primary tumor directly invades adjacent organs, such as the aorta and trachea (stage T4b), or fistula formation is present; ② imaging evidence is present that tumor tissue encases a great vessel by > 90 degrees; ③ endoscopic examination confirms complete or near-complete obstruction of the esophagus, requiring interven-

tional treatment; esophageal or tracheal stent implantation is present; the investigator judges that there is another risk of perforation or a risk of significant gastrointestinal bleeding.

5. Has insufficient bone marrow reserve or hepatic or renal organ function, meeting any one of the following laboratory limits (with no corrective treatment within 1 week before blood collection for laboratory examination):

a) Neutrophil count < 1.5 × 109/L;
b) Platelet count < 90×109/L;
c) Hemoglobin < 90 g/L;
d) Total bilirubin > 1.5 × the upper limit of normal (ULN); if there is confirmed Gilbert syndrome (unconjugated hyperbilirubinemia) or liver metastasis, total bilirubin > 3.0 × ULN;
e) ALT and/or AST > 2.5 × ULN; if liver metastasis is present, then ALT and/or AST > 5.0 × ULN;
f) Creatinine > 1.5 × ULN and creatinine clearance < 50 mL/min (calculated by the Cockcroft-Gault formula in Appendix X); creatinine clearance needs to be confirmed only when creatinine > 1.5 × ULN;
g) International normalized ratio (INR) > 1.5, and activated partial thromboplastin time (APTT) > 1.5 × ULN;
h) Serum albumin (ALB) < 28 g/L.

6. Meeting any one of the following cardiac examination criteria:

a) The average value of the Fridericia-corrected QT interval (QTcF) obtained from an electrocardiogram (ECG) examination at rest is > 470 msec; for the Fridericia formula, see Appendix VII;
b) Resting ECG indicates the presence of any clinically significant rhythm, conduction, or ECG morphological abnormality judged by the investigator to be important (for example, complete left bundle branch block, 3rd-degree atrioventricular block, 2nd-degree atrioventricular block, and PR interval > 250 msec, etc.);
c) There is any factor that increases the risk of QTc prolongation or arrhythmic events, such as heart failure, refractory hypokalemia, congenital long QT syndrome, a family history of long QT syndrome, unexplained sudden death of a first-degree relative under 40 years old,
or any concomitant medication that prolongs the QT interval;
d) Left ventricular ejection fraction (LVEF) < 50%.

7. Has severe, uncontrolled, or active cardiovascular and cerebrovascular disease, including but not limited to:

a) Myocardial infarction (MI) occurring within 6 months before the first administration of the study drug;
b) Unstable angina occurring within 6 months before the first administration;
c) Congestive heart failure (CHF) occurring within 6 months before the first administration;
d) Cerebrovascular accident or transient ischemic attack occurring within 6 months before the first administration.

8. Severe or poorly controlled diabetes, including: diabetic ketoacidosis or hyperglycemic hyperosmolar state occurring within 6 months before the first administration; patients whose glycated hemoglobin test value during the screening period is ≥ 7.5%. (Note: newly diagnosed diabetes is not considered well-controlled diabetes, unless after at least 8 weeks of glucose-lowering treatment and all recorded blood glucose test values during the screening period are < 13.9 mmol/L, including home blood glucose monitoring records, then enrollment is allowed.)

9. Severe or poorly controlled hypertension, including: a previous history of hypertensive crisis or hypertensive encephalopathy; adjustment of antihypertensive drug treatment within 2 weeks before the first administration due to poor blood pressure control; systolic blood pressure ≥ 180 mmHg or diastolic blood pressure ≥ 110 mmHg during the screening period.

10. Having had clinically significant bleeding symptoms or an obvious bleeding tendency within 1 month before the first administration.

11. Having had a severe arterial or venous thrombotic event within 3 months before the first administration, such as deep vein thrombosis, pulmonary embolism, etc. (excluding implanted venous infusion ports, catheter-related thrombosis, or superficial venous thrombosis, as these situations are not regarded as "severe" thromboembolism).

12. Having had a severe infection within 4 weeks before the first administration, including but not limited to infectious complications treated with intravenous antibiotics for ≥ 2 weeks, bacteremia, severe pneumonia, etc.; or having uncontrollable active infection during the screening period. Subjects who are receiving or have received prophylactic antibiotic treatment (such as prevention of urinary tract infection) may be enrolled.

13. Having received continuous glucocorticoid treatment for more than 30 days within 30 days before the first administration, or requiring long-term (≥ 30 days) glucocorticoid treatment (excluding asthma patients requiring long-term inhaled glucocorticoids and patients using topical steroid drugs for the skin), or having other acquired or congenital immunodeficiency diseases, or having a history of organ transplantation (excluding corneal transplanta-

tion).

14. Known to have an active infectious disease, such as active hepatitis B (during the screening period, the viral surface antigen [HBsAg] test result is positive and at the same time the HBV-DNA test value is detected to be ≥ 2 × 103 IU/mL; if, after regular antiviral treatment, repeated testing decreases it to below 2 × 103 IU/mL, enrollment is allowed) or hepatitis C (defined as a positive hepatitis C virus antibody [HCV-Ab] test result during the screening period, and HCV-RNA positive), tuberculosis (evidence of active tuberculosis infection within 1 year), syphilis (both Treponema pallidum-specific antibody and nonspecific antibody are positive), or human immunodeficiency virus HIV infection (anti-HIV antibody positive), etc. Active infectious diseases will not be proactively screened.

15. Currently suffering from hepatic encephalopathy, hepatorenal syndrome, or liver cirrhosis of ≥ Child-Pugh class B.

16. Other moderate to severe pulmonary diseases that may interfere with the detection or management of drug-related pulmonary toxicity and seriously affect respiratory function (including interstitial lung disease, radiation pneumonitis or drug-related pneumonitis requiring steroid treatment, etc., or a history of such diseases).

17. Subjects suffering from active autoimmune disease or having a history of autoimmune disease with a possibility of recurrence (such as systemic lupus erythematosus, inflammatory bowel disease, autoimmune thyroid disease, multiple sclerosis, vasculitis, glomerulonephritis, etc.), or subjects at high risk (such as those who have undergone organ transplantation and need to receive immunosuppressive treatment). However, subjects with the following diseases are allowed to be enrolled: 1) type I diabetes patients whose condition is stable after using a fixed dose of insulin; 2) autoimmune hypothyroidism requiring only hormone replacement therapy; 3) skin diseases not requiring systemic treatment (such as eczema, rash involving less than 10% of the body surface area, psoriasis without ophthalmic symptoms, etc.).

18. A previous history of severe neurological or psychiatric disorders, including conditions interfering with assessment such as epilepsy, dementia, or severe depression.

19. Female patients who are pregnant, breastfeeding, or planning to become pregnant during the study period.

20. Having been vaccinated or having had any degree of allergic or hypersensitivity reaction within 4 weeks before the first administration.

21. Those with a previous history of severe allergy (for example, anaphylactic shock), or who have had a severe infusion reaction, or who are allergic to recombinant human- or murine-derived protein substances.

22. Allergic to any component of the anti-B7H3 antibody-drug conjugate (antibody-conjugated drug, antibody, toxin).

23. Patients who, in the investigator's judgment, may have poor compliance with the procedures and requirements of the study.

24. Patients who, in the investigator's judgment, have any condition that endangers patient safety or interferes with study assessment.

**Endpoint indicators**

1. Primary key indicator and evaluation time

Indicator (efficacy indicator): objective response rate evaluated by the investigator according to the RECIST 1.1 standard

Evaluation time: after the first administration, tumor imaging assessment is performed once every 6 weeks within 24 weeks, and once every 12 weeks starting from Week 25, until the subject has disease progression or withdraws from the trial.

2. Secondary key indicators and evaluation times

Indicator 1 (efficacy indicator): disease control rate (DCR), duration of response (DoR), and progression-free survival (PFS) evaluated by the investigator according to the RECIST 1.1 standard;

Evaluation time: after the first administration, tumor imaging assessment is performed once every 6 weeks within 24 weeks, and once every 12 weeks starting from Week 25, until the subject has disease progression or withdraws from the trial.

Indicator 2 (efficacy indicator): ORR, DCR, DoR, and PFS evaluated by the Independent Review Committee (IRC) according to the RECIST 1.1 standard;

Evaluation time: after the first administration, tumor imaging assessment is performed once every 6 weeks within 24 weeks, and once every 12 weeks starting from Week 25, until the subject has disease progression or withdraws from the trial

Indicator 3 (efficacy indicator + safety indicator): overall survival (OS);

Evaluation time: from the last administration, survival follow-up is conducted once every 12 weeks (± 7 days), until the subject dies or meets other criteria for withdrawal from the trial.
Indicator 4 (safety indicator): safety of injection of the anti-B7H3 antibody-drug conjugate;
Evaluation time: the entire study period, until 90 days after the patient's last administration.
Indicator 5 (safety indicator): pharmacokinetic characteristics of intravenous injection of the anti-B7H3 antibody-drug conjugate;
Evaluation time: the entire study period, until 90 days after the patient's last administration.
Indicator 6 (safety indicator): immunogenicity of intravenous injection of the anti-B7H3 antibody-drug conjugate
Evaluation time: the entire study period, until 90 days after the patient's last administration

**3. Endpoint evaluation and statistical analysis**

3.1. Endpoint evaluation

3.1.1. Efficacy evaluation

3.1.1.1. Evaluation according to RECIST 1.1

At each visit, the investigator will compare baseline and prior imaging evidence, evaluate the subject's tumor treatment response as CR, PR, SD, or PD according to the RECIST 1.1 criteria, and use it to calculate ORR, DCR, DoR, and PFS (see Appendix XI for details). Whether progression of target lesions has occurred is determined by comparison with the lowest tumor burden (that is, the smallest sum of diameters previously recorded). In the absence of progression, comparison will be made with the baseline tumor measurement results to determine whether tumor response (CR, PR) or stable disease (SD) has occurred. For subjects whose tumor status cannot be determined, if there is evidence of progression, it is judged as PD; if there is no evidence of progression, it is judged as not evaluable (NE).

3.1.1.2. Objective response rate (ORR)

Objective response rate is defined as the percentage of subjects who have at least 1 confirmed CR or PR before progression.
Confirmed CR/PR is defined as, 4 weeks after the first evaluation as CR/PR, reassessing the tumor burden again by imaging examination, and the obtained evaluation result, when compared with the previous one, does not indicate tumor progression. The ORR evaluation will include efficacy data until the subject develops PD, while for subjects who do not develop PD, their last evaluable data will be included. However, any CR or PR occurring after termination of study treatment and receipt of other antitumor treatment will not be included in the ORR calculation.

3.1.1.3. Disease control rate (DCR)

Disease control rate is defined as the proportion of subjects whose best overall response is CR, PR, or SD.
In this trial, CR and PR efficacy need to be confirmed; SD must meet the RECIST 1.1 definition and be maintained for at least 5 weeks after start of treatment.

3.1.1.4. Duration of response (DoR)

Duration of response is defined as the time from the date of first meeting the criteria for response to the date of disease progression or death from any cause. The end date of response shall be consistent with the date used to determine the PFS endpoint. The start date of response is defined as the date of the imaging examination that first meets the PR or CR criteria. If the subject has no progression after response, the duration of response will use the censoring time of PFS.

3.1.1.5. Progression-free survival (PFS)

PFS is defined as the time from randomization (if there is no randomization, then from the start of administration of the first dose of study drug) to the occurrence of objective tumor progression or death (whichever event occurs first). In the case where the subject has already discontinued study treatment or received other anticancer therapy before disease progression, it is still necessary to collect the results of the subject's imaging tumor assessments until the occurrence of disease progression as defined by RECIST 1.1.
Subjects who have not progressed or died at the time of analysis will be censored at the date of their last valid imaging examination. If the subject has disease progression or death after missing 2 or more consecutive visits, the subject

will be censored at the date of the last valid imaging examination before the disease progression or death. The derived data for progression-free survival are based on imaging examination dates.

3.1.1.6. Overall survival (OS)

OS is defined as the time from randomization (if there is no randomization, then from the start of administration of the first dose of study drug) to death from any cause. Subjects who have not died at the time of statistical analysis will be censored at the last time point at which the subject is known to be alive.

3.1.2. Safety evaluation

At the end of the study, all safety data will be summarized. All data from the start of treatment until 90 days after the last treatment will be summarized together in the safety evaluation. Safety data before the start of treatment will also be summarized, but will not be included in the AE summary table.
The number of subjects experiencing each AE will be summarized according to the Medical Dictionary for Regulatory Activities (MedDRA) system organ class (SOC), MedDRA preferred term (PT), and CTCAE grade. The number and percentage of subjects experiencing AEs of different categories (for example, relation to study drug or CTCAE $\geq$ 3 grade) will be summarized by different population cohorts. At the same time, adverse events in each category will also be further summarized by different population cohorts through MedDRA system organ class and preferred term. Serious adverse events will be summarized separately.
All safety evaluation items, demographic data, and concomitant medications will be listed one by one by subject and summarized. For death events, detailed listing will be made by subject.

3.1.3. PK evaluation

This study will respectively determine and analyze the blood drug concentrations of the three components of the anti-B7H3 antibody-drug conjugate, including toxin-conjugated antibody, total antibody, and free toxin.
If the data are sufficient, a noncompartmental model will be used to calculate the pharmacokinetic parameters of the subjects, including but not limited to time to peak concentration (Tmax), peak concentration (Cmax), area under the concentration-time curve from time 0 to the last measurable concentration time point t (AUC0-t), area under the concentration-time curve from time 0 to infinity (AUC0-$\infty$), elimination halflife (t1/2), steady-state peak concentration (Css, max), steady-state trough concentration (Css, min), steady-state area under the concentration-time curve (AUCss), clearance (CL), steady-state apparent volume of distribution (Vss), accumulation ratio (Rac), etc., and descriptive statistical summaries and listings of the pharmacokinetic parameters will be made by dose group.

3.1.4. Immunogenicity evaluation

This study will conduct detection of antibodies against the anti-B7H3 antibody-drug conjugate (ADA) in immunogenicity samples, and when necessary conduct neutralizing antibody (Nab) activity detection, in order to evaluate the impact of ADA on drug PK, safety, and efficacy.

3.1.5. Exploratory endpoint evaluation

The results of the exploratory studies may or may not present this part of the data in the study summary report. These data may also be subjected to comprehensive analysis in combination with other study data after completion of the study.

3.1.5.1. Evaluation of the correlation between baseline B7-H3 protein expression level and efficacy

This study will detect the B7-H3 protein expression level in tumor tissue samples of subjects, and evaluate the relationship between B7-H3 protein expression and efficacy.

3.1.5.2. Evaluation of the relationship between baseline gene abnormal status and efficacy

This study will detect gene abnormal status in blood samples of subjects, and evaluate the relationship between baseline gene abnormalities and drug efficacy.

3.1.5.3. Evaluation of the relationship between baseline tumor tissue PD-L1 protein expression and efficacy

This study will detect the PD-L1 protein expression level in tumor samples of subjects, and evaluate the relationship between PD-L1 protein expression and drug efficacy.

3.1.5.4. sB7-H3 biomarker evaluation

This study will detect the concentration of sB7-H3 in blood samples of subjects, and evaluate the relationship between sB7-H3 and tumor cell B7-H3 expression as well as the relationship between sB7-H3 and drug efficacy.

3.1.5.5. Relationship between exposure and clinical response (efficacy and safety)

This study will detect the concentration of the study drug in blood samples of subjects, and evaluate the relationship between exposure and efficacy and safety.

3.2. Statistical methods

All statistical analyses will use SAS 9.4 and higher versions of statistical analysis software. The statistical analysis methods for the data collected in this study will be included in the Statistical Analysis Plan (SAP), finalized and filed by the sponsor. Any modification to this study protocol, if judged by the sponsor or the principal investigator to have a relatively large impact on the statistical analysis plan, requires the SAP to be revised again so as to remain consistent with the study protocol.

3.2.1. Statistical analysis sets

Safety Set (SS): all subjects enrolled and having used the investigational drug at least once.

Efficacy Evaluable Set (EES): all subjects enrolled and having used the investigational drug at least once, with baseline and at least one post-baseline tumor efficacy assessment. The Efficacy Evaluable Set is the primary analysis set for efficacy analysis.

Pharmacokinetic Set (PKS): includes all enrolled subjects who have received the investigational drug and have at least one measurable post-dose blood drug concentration.

Immunogenicity Analysis Set (ADAS): all subjects enrolled and having used the investigational drug at least once, with baseline and at least one post-baseline immunogenicity evaluation datum.

3.2.2. General rules for statistical analysis

For the subject data in the phase IIa dose-expansion stage, analysis will be conducted separately for each expansion cohort.

Unless otherwise specified, ORR, DCR, DoR, and PFS will be summarized according to IRC and investigator assessments.

Trial data will mainly be analyzed using descriptive statistical methods. For measurement data, the number of observations, mean, standard deviation, median, maximum value, and minimum value will generally be listed. For count data, frequency and rate (constituent ratio) will be listed; when the frequency is 0, the rate (constituent ratio) will not be displayed. Interval estimates of parameters all use two-sided 95% confidence intervals.

3.2.3. Efficacy analysis

Efficacy indicators will be analyzed using the results assessed by the investigator/IRC according to the RECIST 1.1 standard. The efficacy analysis will be based on the Efficacy Evaluable Set. The efficacy data of subjects in the phase IIa dose-expansion stage will be analyzed for efficacy separately according to population cohorts.

3.2.3.1. Objective response rate

Tumor response data (CR, PR, SD, PD, and NE) and ORR will be summarized according to different population cohorts, and 95% confidence intervals will be calculated (Clopper-Pearson method).

3.2.3.2. Disease control rate

Disease control rate will be summarized according to different population cohorts, and 95% confidence intervals will be calculated (Clopper-Pearson method).

3.2.3.3. Progression-free survival

Progression-free survival will be summarized according to different population cohorts, Kaplan-Meier plots for different population cohorts will be drawn, and the median PFS and its 95% confidence interval will be estimated.

3.2.3.4. Duration of response

Duration of response will be summarized according to different population cohorts, Kaplan-Meier plots for different population cohorts will be drawn, and the median DoR of each group and its 95% confidence interval will be estimated.

3.2.3.5. Overall survival

Overall survival will be summarized according to different population cohorts, Kaplan-Meier plots for different population cohorts will be drawn, and the median OS and its 95% confidence interval will be estimated.

3.2.4. Safety analysis

3.2.4.1. Adverse events

Based on the Safety Set, AE, laboratory parameter data, vital sign data, electrocardiogram data, and the like will be subjected to descriptive statistical summary and listing. AE occurring from the start of treatment until 90 days after the last treatment will be summarized in the safety evaluation.

All adverse events will be summarized for each dose group, mainly including:

1) the number and proportion of subjects with at least one AE;
2) the number and proportion of subjects with at least one AE related to the study drug;
3) the number and proportion of subjects with at least one higher-grade severe event (CTCAE ≥ Grade 3);
4) the number and proportion of subjects with at least one higher-grade severe event (CTCAE ≥ Grade 3) related to the study drug;
5) the number and proportion of subjects with at least one SAE;
6) the number and proportion of subjects with at least one SAE related to the study drug;
7) the number and proportion of subjects with dose adjustment (interrupted dosing, delayed dosing, and dose reduction) due to AE;
8) the number and proportion of subjects with treatment termination due to AE;
9) the number and proportion of subjects with death due to AE.

Adverse events will be summarized by system organ class and preferred term. All adverse events and serious adverse events will be listed.

3.2.4.2. Laboratory examinations

For examination items in laboratory examinations that are quantitative data in nature, such as hematological examinations, urine examinations, blood biochemistry examinations, and the like, the baseline data, post-dosing data, and post-dosing change data will be summarized by each follow-up visit and each treatment group; for qualitative data such as whether each examination item is normal and whether it has clinical significance, the changes in clinical significance from baseline to each post-dosing follow-up visit will be summarized.

3.2.4.3. Body weight and vital signs

Body weight and vital signs are quantitative data in nature, and the baseline data, post-dosing data, and post-dosing change data will be summarized by each follow-up visit and each treatment group; for qualitative data such as whether each examination item is normal and whether it has clinical significance, the changes from baseline to each post-

dosing follow-up visit will be described in the form of cross tables.

3.2.4.4. ECG analysis

Mean ± standard deviation, maximum value, minimum value, and median will be used to describe the measured values and the changes from baseline of indicators such as heart rate, PR interval, QT interval, and QTc interval before and after treatment, and changes of clinical significance before and after treatment will be summarized.

For baseline and post-treatment QTc interval abnormalities, summaries will respectively be made according to the proportions of post-treatment (maximum post-treatment value) QTcF ≤ 450 ms, > 450 ms and ≤ 480 ms, > 480 ms and ≤ 500 ms, and > 500 ms, and the changes from baseline after treatment (maximum post-treatment value) will be summarized according to the proportions of ≤ 30 ms, 30~≤ 60 ms, and > 60 ms.

3.2.4.5. ECOG PS score

The changes in ECOG PS score at each follow-up visit relative to baseline will be summarized according to different population cohorts.

3.2.5. PK analysis

Drug concentration and pharmacokinetic parameter analyses will be based on the PKS, and will be analyzed separately according to the single-dose stage and the multiple-dose stage.

3.2.5.1. Drug concentration analysis

Drug concentration analysis will be based on the PKS.

Descriptive statistical analysis will be performed on the sample concentrations of the blood drug concentrations of the test substance anti-B7H3 antibody-drug conjugate according to dose group, cycle, date, and planned sampling time point, respectively. These include the number of cases, arithmetic mean, standard deviation, median, coefficient of variation, minimum value, maximum value, geometric mean, geometric standard deviation, and geometric coefficient of variation, and the like.

According to dose group, cycle and date, and actual sampling time points, individual blood drug concentration curves and corresponding semi-logarithmic c-t curves will be plotted, and at planned time points, geometric mean c-t curves and corresponding semi-logarithmic c-t curves will be plotted.

3.2.5.2. Pharmacokinetic parameter analysis

Pharmacokinetic parameter analysis will be based on the PKS.

If the data are sufficient, Phoenix WinNonlin software (version 8.0 and above) will be used to calculate the PK parameters for first-dose administration and multiple-dose administration, respectively, and the first-dose PK parameters include but are not limited to: Cmax, Tmax, t1/2, AUC0-t, AUC0-∞, CL, and Vz. The multiple-dose PK parameters include but are not limited to: steady-state peak concentration (Css,max), steady-state trough concentration (Css,min), steady-state time to peak concentration (Tss, max), steady-state area under the plasma concentration-time curve (AUCss), steady-state plasma clearance (CLss), and steady-state volume of distribution (Vss), etc.

The PK parameters of the test substance anti-B7H3 antibody-drug conjugate will be subjected to descriptive statistical analysis according to dose group and cycle, respectively. These include number of cases, arithmetic mean, standard deviation, median, minimum value, maximum value, coefficient of variation, geometric mean, geometric standard deviation, and geometric coefficient of variation, etc.

3.2.5.3. Exposure-response analysis

Where the data permit, exploratory analysis of the exposure-response relationship of the analytes of the anti-B7H3 antibody-drug conjugate may be appropriately carried out.

3.2.6. Immunogenicity analysis

Immunogenicity analysis will be based on the immunogenicity analysis set, and all anti-drug antibody (immunogenicity) test results will be listed. The proportion of subjects testing positive for immunogenicity, the time of first positive immunogenicity test in subjects, the duration of positive immunogenicity test in subjects, and the like will be

analyzed. If the data permit, in order to explore the potential relationship among immunogenicity, drug trough concentration, safety, and efficacy, the incidence and types of AE may be explored according to the overall drug trough concentration/immunogenicity situation, and the exploratory analysis results will be presented separately.

3.2.7. Analysis of the relationship between baseline tumor tissue B7-H3 protein expression and efficacy

Stratified analysis of efficacy indicators will be conducted according to different population cohorts and B7-H3 protein expression levels, to evaluate the relationship between B7-H3 protein expression and efficacy.

3.2.8. Analysis of the relationship between baseline gene abnormal status and efficacy

Stratified analysis of efficacy indicators will be conducted according to different population cohorts and different gene abnormal statuses, to evaluate the relationship between gene abnormal status and efficacy.

3.2.9. Analysis of the relationship between baseline tumor tissue PD-L1 protein expression and efficacy

Stratified analysis of efficacy indicators will be conducted according to different population cohorts and tumor tissue PD-L1 protein expression levels, to evaluate the relationship between PD-L1 protein expression and efficacy.

3.2.10. Analysis of the relationship between baseline sB7-H3 level and baseline tumor tissue B7-H3 protein expression level

Stratified analysis of tumor tissue B7-H3 protein expression levels will be conducted according to different population cohorts and sB7-H3 levels, to evaluate the relationship between sB7-H3 level and tumor tissue B7-H3 protein expression level.

3.2.11. Analysis of the relationship between sB7-H3 level and efficacy

Stratified analysis of efficacy indicators will be conducted according to different population cohorts and sB7-H3 levels, to evaluate the relationship between sB7-H3 level and efficacy.

3.2.12. Analysis of the relationship between exposure and clinical response (efficacy and safety)

If the data permit, PK analysis will be conducted on all subjects, to evaluate the relationship between exposure and efficacy and safety.

**III. Test results**

**1. Efficacy**

As of June 25, 2024 the interim study results show that 4 ESCC subjects enrolled in phase Ib received 10 mg/kg dosing, and 22 ESCC subjects enrolled in phase II received 8 mg/kg dosing, the baseline characteristics of the subjects are shown in Table 3, all subjects had squamous cell carcinoma as the pathological type, and all had previously received immunotherapy and chemotherapy and the number of prior lines of therapy did not exceed 3. Wherein.

Table 3

| | | **8.0 mg/kg (N=22)** | **10.0 mg/kg (N=4)** |
|---|---|---|---|
| **Age (years)** | Mean (standard deviation) | 62.7 (6.71) | 55.0 (2.58) |
| | Median (q1, q3) | 64.0 (57.0, 68.0) | 55.0 (53.0, 57.0) |
| **Height (cm)** | Mean (standard deviation) | 169.68 (5.524) | 170.00 (8.446) |
| | Median (q1, q3) | 170.50 (167.00, 173.00) | 171.50 (163.50, 176.50) |
| **Body weight (kg)** | Mean (standard deviation) | 60.01 (10.230) | 50.30 (6.916) |
| | Median (q1, q3) | 58.70 (53.10, 67.10) | 49.60 (45.00, 55.60) |
| **BMI (kg/m2)** | Mean (standard deviation) | 20.81 (3.243) | 17.33 (0.862) |

(continued)

| | | 8.0 mg/kg (N=22) | 10.0 mg/kg (N=4) |
|---|---|---|---|
| | Median (q1, q3) | 20.90 (18.30, 22.90) | 17.00 (16.85, 17.80) |
| **Sex** | Male (%) | 19 (86.4) | 4 (100.0) |
| | Female (%) | 3 (13.6) | 0 |
| **ECOG score** | 0 (%) | 4 (18.2) | 0 |
| | 1 (%) | 18 (81.8) | 4 (100.0) |
| **Pathological type at study entry** | Squamous cell carcinoma (%) | 22 (100.0) | 4 (100.0) |
| **Tumor stage at study entry** | III (%) | 2 (9.1) | 0 |
| | IV (%) | 14 (63.6) | 3 (75.0) |
| | IVA (%) | 0 | 1 (25.0) |
| | IVB (%) | 6 (27.3) | 0 |
| **History of prior radiotherapy** | Yes (%) | 10 (45.5) | 1 (25.0) |
| | No (%) | 12 (54.5) | 3 (75.0) |
| **Number of prior lines of systemic antitumor drug treatment** | Mean (standard deviation) | 1.2 (0.53) | 1.5 (1.00) |
| | Median (minimum, maximum) | 1.0 (1.0, 1.0) | 1.0 (1.0, 2.0) |
| **Types of prior systemic antitumor treatment** | Chemotherapy (%) | 22 (100.0) | 4 (100.0) |
| | Immunotherapy (%) | 21 (95.5) | 4 (100.0) |
| | Targeted therapy (%) | 5 (22.7) | 0 |
| **Baseline liver metastasis** | Yes (%) | 6 (27.3) | 1 (25.0) |
| | No (%) | 16 (72.7) | 3 (75.0) |

There were a total of 12 evaluable patients in the 8 mg/kg dose group, and a total of 3 efficacy-evaluable patients in the 10 mg/kg dose group. As shown by the efficacy results in Table 4, the ORR of the 8 mg/kg dose group was 16.7% (2/12, including 1 confirmed response), and the DCR was 75% (9/12); the ORR of the 10 mg/kg dose group was 66.7% (2/3, of which 1 case was confirmed), and the DCR was 100% (3/3). The ORR of existing standard treatments such as irinotecan, paclitaxel, and docetaxel is about 6%-10%. The anti-B7H3 antibody-drug conjugate demonstrated better antitumor activity in esophageal squamous cell carcinoma than existing standard therapies.

Table 4

| | 8.0 mg/kg (N=12) | 10.0 mg/kg (N=3) |
|---|---|---|
| CR (n) | 0 | 0 |
| PR (n) | 2 | 2 |
| SD (n) | 7 | 1 |
| PD (n) | 3 | 0 |
| ORR (%) | 16.7 | 66.7 |
| DCR (%) | 75 | 100.0 |

**2. Safety**

As of June 25, 2024, the phase I and phase II safety results are as shown in Table 5, and the safety data show that the anti-B7H3 antibody-drug conjugate has good safety and tolerability, especially in the 8.0 mg/kg dose group, where the incidence of any treatment-emergent adverse event (TEAE), the incidence of treatment-emergent adverse events (TEAE)

leading to dose reduction, the incidence of treatment-emergent adverse events (TEAE) leading to permanent discontinuation, and the incidence of interstitial lung disease adverse events (ILD) related to the study drug were all lower than those of similar drugs, suggesting an improved safety profile.

Table 5

| | 1.0 mg/kg (N=3) n (%) | 2.0 mg/kg (N=1) n (%) | 4.0 mg/kg (N=3) n (%) | 6.0 mg/kg (N=3) n (%) | 8.0 mg/kg (N=285) n (%) | 10.0 mg/kg (N=113) n (%) |
|---|---|---|---|---|---|---|
| Any adverse event | 3 (100.0) | 1 (100.0) | 3 (100.0) | 3 (100.0) | 277 (97.2) | 113 (100.0) |
| Adverse events leading to dose reduction | 0 | 0 | 0 | 0 | 27 (9.5) | 43 (38.1) |
| Adverse events leading to permanent discontinuation | 0 | 0 | 0 | 0 | 11 (3.9) | 10 (8.8) |
| Interstitial lung disease adverse events related to the study drug | 0 | 0 | 0 | 0 | 7 (2.5) | 3 (2.7) |

References

1. Chapoval AI, Ni J, Lau JS, et al. B7-H3: A costimulatory molecule for T cell activation and IFN-gamma production. Nat Immunol. 2001, 2(3): 269-274.
2. Wang L, Kang FB, Shan BE. B7-H3-mediated tumor immunology: Friend or foe? Int J Cancer. 2014, 134(12): 2764-2771.
3. Zhang G, Hou J, Shi J, et al. Soluble CD276 (B7-H3) is released from monocytes, dendritic cells and activated T cells and is detectable in normal human serum. Immunology. 2008, 123(4): 538-546.
4. Li G, Quan YC, Che FY, et al. B7-H3 in tumors: friend or foe for tumor immunity? Cancer Chemother Pharmacol. 2018, 81(2): 245-253.
5. Wang L, Zhang Q, Chen W, et al. B7-H3 is overexpressed in patients suffering osteosarcoma and associated with tumor aggressiveness and metastasis. PLoS One. 2013;8(8):e70689. Published 2013 Aug 5
6. Picarda E, Ohaegbulam KC, Zang X. Molecular Pathways: Targeting B7-H3 (CD276) for Human Cancer Immunotherapy. Clin Cancer Res. 2016;22(14):3425-3431
7. Hu J, Jiang C, Zheng M, et al. Overexpression of B7-H3 as an opportunity for targeted therapy in head and neck cancers. Am J Transl Res. 2019;11(8):5183-5196. Published 2019 Aug 15.
8. Lin W, Xu Y, Gao J, et al. Multi-Omics Data Analyses Identify B7-H3 as a Novel Prognostic Biomarker and Predict Response to Immune Checkpoint Blockade in Head and Neck Squamous Cell Carcinoma. Front Immunol. 2021;12:757047. Published 2021 Oct 5.
9. Altan, M., Pelekanou, V., Schalper, K. A., Toki, M., Gaule, P., Syrigos, K., Herbst, R. S., & Rimm, D. L. (2017). B7-H3 Expression in NSCLC and Its Association with B7-H4, PD-L1 and Tumor-Infiltrating Lymphocytes. Clinical cancer research: an official journal of the American Association for Cancer Research, 23(17), 5202-5209.
10. Zhou, WT., Jin, WL. B7-H3/CD276: An Emerging Cancer Immunotherapy. Front Immunol. 2021;12: 701006.
11. Maruki Y, Takashima A, Miyamoto T, et al. Expression of B7-H3 (CD276) in surgically resected esophageal squamous cell carcinoma. Journal of Clinical Oncology. 2018/02/01 2018;36(4_suppl):70-70.
12. Song J, Shi W, Zhang Y, Sun M, Liang X, Zheng S. Epidermal growth factor receptor and B7-H3 expression in esophageal squamous tissues correlate to patient prognosis. Onco Targets Ther. 2016;9:6257-6263. Published 2016 Oct 12.
13. Siegel RL, Miller KD, Fuchs HE, Jemal A. Cancer statistics, 2022. CA Cancer J Clin. 2022;72(1):7-33.
14. Morgan E, Soerjomataram I, Rumgay H, et al. The global landscape of esophageal squamous cell carcinoma and esophageal adenocarcinoma incidence and mortality in 2020 and projections to 2040: New estimates from GLOBOCAN 2020 [published online ahead of print, 2022 Jun 4]. Gastroenterology. 2022;S0016-5085(22)00608-4.
15. Thrift AP. Global burden and epidemiology of Barrett oesophagus and oesophageal cancer.Nat Rev Gastroenterol Hepatol. 2021;18(6):432-443.
16. Sun JM, et al. Pembrolizumab plus chemotherapy versus chemotherapy alone for first-line treatment of advanced oesophageal cancer (KEYNOTE-590): a randomised, placebo-controlled, phase 3 study [published correction appears in Lancet. 2021;398(10302):759-771.

17. Luo H, Lu J, Bai Y, et al. Effect of Camrelizumab vs Placebo Added to Chemotherapy on Survival and Progression-Free Survival in Patients With Advanced or Metastatic Esophageal Squamous Cell Carcinoma: The ESCORT-1st Randomized Clinical Trial. JAMA. 2021;326(10):916-925.
18. Doki Y, Ajani JA, Kato K, et al. Nivolumab Combination Therapy in Advanced Esophageal Squamous-Cell Carcinoma. N Engl J Med. 2022;386(5):449-462.
19. Wang ZX, Cui C, Yao J, et al. Toripalimab plus chemotherapy in treatmentnaïve, advanced esophageal squamous cell carcinoma (JUPITER-06): A multi-center phase 3 trial. Cancer Cell. 2022;40(3):277-288.e3.
20. Huang J, Xu J, Chen Y, et al. Camrelizumab versus investigator's choice ofchemotherapy as second-line therapy for advanced or metastatic oesophageal squamous cell carcinoma (ESCORT): a multicentre, randomised, open-label, phase 3 study. Lancet Oncol. 2020;21(6):832-842.
21. Kojima T, Shah MA, Muro K, et al. Randomized Phase III KEYNOTE-181 Study of Pembrolizumab Versus Chemotherapy in Advanced Esophageal Cancer. J Clin Oncol. 2020;38(35):4138-4148.

All references cited herein are incorporated by reference, as if each individual publication, patent application, or patent were specifically and individually indicated to be incorporated by reference. The citation of references herein is not intended as an admission that the references are relevant prior art, nor does it constitute any admission regarding the content or date of these publications or documents. To the extent that the definition of a term in a statement provided in the references conflicts with the definition provided in the specification of the present invention, the definition provided in the specification of the present invention shall be used to interpret the claimed invention.

## Claims

**1.** A method for preventing and/or treating cancer using an antibody-drug conjugate, comprising administering to a patient a therapeutically effective amount of the antibody-drug conjugate, a pharmaceutically acceptable salt, stereoisomer, or metabolite thereof, or a solvate of any of the foregoing, wherein the structure of the antibody-drug conjugate is as shown in formula (I):

(I)

wherein:

n is 1 to 10, preferably 2 to 8, more preferably 3 to 8, and n is a decimal or an integer;
Pc is an anti-B7H3 antibody or an antigen-binding fragment thereof.

**2.** The method according to claim 1, wherein the anti-B7H3 antibody or the antigen-binding fragment thereof comprises: heavy-chain HCDR1, HCDR2, and HCDR3 respectively as shown in amino acid sequences of SEQ ID NO: 01, 02, and 03, and light-chain LCDR1, LCDR2, and LCDR3 respectively as shown in amino acid sequences of SEQ ID NO: 04, 05, and 06.

**3.** The method according to claim 1 or 2, wherein the anti-B7H3 antibody or the antigen-binding fragment thereof is selected from a humanized antibody or a fragment thereof.

**4.** The method according to claim 3, wherein the anti-B7H3 antibody or the antigen-binding fragment thereof comprises a heavy-chain constant region of the human IgG1, IgG2, IgG3, or IgG4 isotype, and comprises a light-chain constant region of $\kappa$ or $\lambda$; preferably, the anti-B7H3 antibody or the antigen-binding fragment thereof comprises a heavy-chain constant region of the IgG1 or IgG4 isotype.

5. The method according to claim 3, wherein the heavy-chain variable region sequence of the anti-B7H3 antibody or the antigen-binding fragment thereof is the sequence as shown in SEQ ID NO: 07 or a variant thereof, and the light-chain variable region sequence is the sequence as shown in SEQ ID NO: 08 or a variant thereof.

6. The method according to any one of claims 1 to 5, wherein the heavy-chain sequence of the anti-B7H3 antibody or the antigen-binding fragment thereof is the sequence as shown in SEQ ID NO: 09 or a variant thereof, and the light-chain sequence is the sequence as shown in SEQ ID NO: 10 or a variant thereof.

7. The method according to any one of claims 1 to 6, wherein the route of administration of the antibody-drug conjugate, a pharmaceutically acceptable salt, stereoisomer, or metabolite thereof, or a solvate of any of the foregoing, is oral administration, parenteral administration, or transdermal administration, and the parenteral administration mode is selected from intravenous injection, intravenous infusion, subcutaneous injection, and intramuscular injection, preferably intravenous infusion.

8. The method according to claim 7, wherein the antibody-drug conjugate is administered to the patient by intravenous infusion once every week, once every two weeks, once every three weeks, once every four weeks, or once every six weeks, preferably once every three weeks.

9. The method according to claim 8, wherein the administered dose of the antibody-drug conjugate, a pharmaceutically acceptable salt, stereoisomer, or metabolite thereof, or a solvate of any of the foregoing, is 0.1 mg/kg to 20 mg/kg, preferably 0.5 mg/kg to 18 mg/kg, more preferably 1 mg/kg to 16 mg/kg, and further preferably 4 mg/kg to 12 mg/kg.

10. The method according to claim 9, wherein the administered dose of the antibody-drug conjugate, a pharmaceutically acceptable salt, stereoisomer, or metabolite thereof, or a solvate of any of the foregoing, is selected from 4.0 mg/kg, 4.1 mg/kg, 4.2 mg/kg, 4.3 mg/kg, 4.4 mg/kg, 4.5 mg/kg, 4.6 mg/kg, 4.7 mg/kg, 4.8 mg/kg, 4.9 mg/kg, 5 mg/kg, 5.1 mg/kg, 5.2 mg/kg, 5.3 mg/kg, 5.4 mg/kg, 5.5 mg/kg, 5.6 mg/kg, 5.7 mg/kg, 5.8 mg/kg, 5.9 mg/kg, 6.0 mg/kg, 6.1 mg/kg, 6.2 mg/kg, 6.3 mg/kg, 6.4 mg/kg, 6.5 mg/kg, 6.6 mg/kg, 6.7 mg/kg, 6.8 mg/kg, 6.9 mg/kg, 7 mg/kg, 7.1 mg/kg, 7.2 mg/kg, 7.3 mg/kg, 7.4 mg/kg, 7.5 mg/kg, 7.6 mg/kg, 7.7 mg/kg, 7.8 mg/kg, 7.9 mg/kg, 8 mg/kg, 8.1 mg/kg, 8.2 mg/kg, 8.3 mg/kg, 8.4 mg/kg, 8.5 mg/kg, 8.6 mg/kg, 8.7 mg/kg, 8.8 mg/kg, 8.9 mg/kg, 9 mg/kg, 9.1 mg/kg, 9.2 mg/kg, 9.3 mg/kg, 9.4 mg/kg, 9.5 mg/kg, 9.6 mg/kg, 9.7 mg/kg, 9.8 mg/kg, 9.9 mg/kg, 10 mg/kg, 10.1 mg/kg, 10.2 mg/kg, 10.3 mg/kg, 10.4 mg/kg, 10.5 mg/kg, 10.6 mg/kg, 10.7 mg/kg, 10.8 mg/kg, 10.9 mg/kg, 11 mg/kg, 11.1 mg/kg, 11.2 mg/kg, 11.3 mg/kg, 11.4 mg/kg, 11.5 mg/kg, 11.6 mg/kg, 11.7 mg/kg, 11.8 mg/kg, 11.9 mg/kg, or 12 mg/kg.

11. The method according to any one of claims 7-10, wherein 8 mg/kg or 10 mg/kg of the antibody-drug conjugate, a pharmaceutically acceptable salt, stereoisomer, or metabolite thereof, or a solvate of any of the foregoing, is administered to the patient by intravenous infusion once every three weeks.

12. The method according to any one of claims 1 to 11, wherein the cancer is selected from breast cancer, ovarian cancer, cervical cancer, lung cancer, uterine cancer, prostate cancer, kidney cancer, urethral cancer, bladder cancer, liver cancer, gastric cancer, salivary gland cancer, esophageal cancer, melanoma, glioma, neuroblastoma, sarcoma, head and neck cancer, colorectal cancer, leukemia, bone cancer, skin cancer, thyroid cancer, pancreatic cancer, and lymphoma; the prostate cancer is preferably castration-resistant prostate cancer, the head and neck cancer is preferably head and neck squamous cell carcinoma, and the esophageal cancer is preferably esophageal squamous cell carcinoma, esophageal adenocarcinoma, or adenocarcinoma of the esophagogastric junction.

13. The method according to any one of claims 1 to 12, wherein the patient is a pathologically confirmed patient with unresectable locally advanced, recurrent, or metastatic cancer, or another advanced solid tumor patient, preferably a patient with unresectable locally advanced, recurrent, or metastatic cancer who has progressed after or is intolerant to at least first-line treatment.

14. The method according to claim 13, wherein the patient is a patient with unresectable locally advanced, recurrent, or metastatic esophageal squamous cell carcinoma who has progressed after or is intolerant to at least first-line treatment; and/or a patient with unresectable locally advanced, recurrent, or metastatic esophageal adenocarcinoma or adenocarcinoma of the esophagogastric junction who has progressed after or is intolerant to at least first-line treatment.

15. The method according to claim 14, wherein the unresectable locally advanced or recurrent esophageal squamous cell carcinoma patient who has progressed after or is intolerant to at least first-line treatment is an unresectable locally

advanced or locally recurrent esophageal squamous cell carcinoma patient who needs to have received, at the locally advanced or recurrent stage, at least (radical) concurrent chemoradiotherapy or sequential chemoradiotherapy; and the metastatic esophageal squamous cell carcinoma patient who has progressed after or is intolerant to at least first-line treatment is a metastatic esophageal squamous cell carcinoma patient who has received at least platinum-containing chemotherapy.

**16.** The method according to claim 14, wherein the unresectable locally advanced or locally recurrent esophageal adenocarcinoma or adenocarcinoma of the esophagogastric junction patient needs to have received at least (radical) concurrent chemoradiotherapy or sequential chemoradiotherapy.

**17.** The method according to claim 14, wherein the metastatic esophageal adenocarcinoma or adenocarcinoma of the esophagogastric junction patient is a HER-2-positive metastatic esophageal adenocarcinoma or adenocarcinoma of the esophagogastric junction patient or a HER-2-negative metastatic esophageal adenocarcinoma or adenocarcinoma of the esophagogastric junction patient.

**18.** The method according to claim 17, wherein the HER-2-positive metastatic esophageal adenocarcinoma or adenocarcinoma of the esophagogastric junction patient has received at least anti-HER-2 treatment combined with platinum-containing chemotherapy; and the HER-2-negative metastatic esophageal adenocarcinoma or adenocarcinoma of the esophagogastric junction patient has received at least platinum-containing chemotherapy.

**19.** The method according to claim 13, wherein said other advanced solid tumor patient is an advanced solid tumor patient for whom there is no standard treatment regimen or who has failed standard treatment.

**20.** The method according to any one of claims 1-19, wherein the patient is selected based on an elevated level of a biomarker related to cancer.

**21.** The method according to any one of claims 1-20, wherein evaluation of the therapeutic effect comprises detecting the concentration of a biomarker in a patient sample, and preferably, the biomarker is selected from soluble B7-H3 and PD-L1.

**22.** The method according to claim 21, wherein by determining the soluble B7-H3 concentration detected from patient blood samples collected during the screening period, during treatment, and at the end-of-treatment visit and/or by determining the PD-L1 protein expression in patient tumor tissue samples, the relationship between the biomarker and the efficacy of the antibody-drug conjugate is evaluated.

**23.** Use of an antibody-drug conjugate, a pharmaceutically acceptable salt, stereoisomer, or metabolite thereof, or a solvate of any of the foregoing, in the preparation of a medicine for preventing/treating cancer, wherein the structure of the antibody-drug conjugate is as shown in formula (I):

(I)

wherein:

n is 1 to 10, preferably 2 to 8, more preferably 3 to 8, and n is a decimal or an integer;
Pc is an anti-B7H3 antibody or an antigen-binding fragment thereof;
the cancer is selected from breast cancer, ovarian cancer, cervical cancer, lung cancer, uterine cancer, prostate cancer, kidney cancer, urethral cancer, bladder cancer, liver cancer, gastric cancer, salivary gland cancer, esophageal cancer, melanoma, glioma, neuroblastoma, sarcoma, head and neck cancer, colorectal cancer,

leukemia, bone cancer, skin cancer, thyroid cancer, pancreatic cancer, and lymphoma; the prostate cancer is preferably castration-resistant prostate cancer, the head and neck cancer is preferably head and neck squamous cell carcinoma, and the esophageal cancer is preferably esophageal squamous cell carcinoma, esophageal adenocarcinoma, or adenocarcinoma of the esophagogastric junction.

**24.** The use of the antibody-drug conjugate according to claim 23 in the preparation of a medicine for treating cancer, wherein the anti-B7H3 antibody or the antigen-binding fragment thereof comprises: heavy-chain HCDR1, HCDR2, and HCDR3 respectively as shown in the amino acid sequences of SEQ ID NO: 01, 02, and 03, and light-chain LCDR1, LCDR2, and LCDR3 respectively as shown in the amino acid sequences of SEQ ID NO: 04, 05, and 06.

**25.** The use of the antibody-drug conjugate according to claim 22 or 23 in the preparation of a medicine for treating cancer, wherein the anti-B7H3 antibody or the antigen-binding fragment thereof is selected from a humanized antibody or a fragment thereof.

**26.** The use of the antibody-drug conjugate according to claim 25 in the preparation of a medicine for treating cancer, wherein the anti-B7H3 antibody or the antigen-binding fragment thereof comprises a heavy-chain constant region of the human IgG1, IgG2, IgG3, or IgG4 isotype, and comprises a light-chain constant region of κ or λ; preferably, the anti-B7H3 antibody or the antigen-binding fragment thereof comprises a heavy-chain constant region of the IgG1 or IgG4 isotype.

**27.** The use of the antibody-drug conjugate according to claim 25 in the preparation of a medicine for treating cancer, wherein the heavy-chain variable region sequence of the anti-B7H3 antibody or the antigen-binding fragment thereof is as shown in SEQ ID NO: 07, and the light-chain variable region sequence is as shown in SEQ ID NO: 08.

**28.** The use of the antibody-drug conjugate according to any one of claims 23-27 in the preparation of a medicine for treating cancer, wherein the heavy-chain sequence of the anti-B7H3 antibody or the antigen-binding fragment thereof is as shown in SEQ ID NO: 09, and the light-chain sequence is as shown in SEQ ID NO: 10.

**29.** The use according to any one of claims 23-28, wherein the prevention and/or treatment comprises administering to a patient a therapeutically effective amount of the anti-B7H3 antibody-drug conjugate of formula (I), a pharmaceutically acceptable salt, stereoisomer, or metabolite thereof, or a solvate of any of the foregoing, and the route of administration is oral administration, parenteral administration, or transdermal administration, and the parenteral administration mode is selected from intravenous injection, intravenous infusion, subcutaneous injection, and intramuscular injection, preferably intravenous infusion.

**30.** The use according to claim 29, wherein the antibody-drug conjugate is administered to the patient by intravenous infusion once every week, once every two weeks, once every three weeks, once every four weeks, or once every six weeks, preferably once every three weeks.

**31.** The use according to any one of claims 23-30, wherein the prevention and/or treatment comprises administering to a patient a therapeutically effective amount of the anti-B7H3 antibody-drug conjugate of formula (I), a pharmaceutically acceptable salt, stereoisomer, or metabolite thereof, or a solvate of any of the foregoing, at a dose of 0.5-18 mg/kg, preferably at a dose of 1-16 mg/kg, more preferably at a dose of 4-12 mg/kg, and still more preferably at a dose of 8 mg/kg or 10 mg/kg.

**32.** The use according to any one of claims 23-31, wherein the prevention and/or treatment comprises administering to a patient a therapeutically effective amount of the anti-B7H3 antibody-drug conjugate of formula (I) or a pharmaceutically acceptable salt, stereoisomer, metabolite, or solvate compound thereof, and the route of administration is oral administration, parenteral administration, or transdermal administration, and the parenteral administration is selected from intravenous injection, intravenous infusion, subcutaneous injection, and intramuscular injection, preferably intravenous infusion.

ES0136 PDX
G1, B, 5 mg/kg, i.v, QW×3 times
G2, A, 5 mg/kg, i.v, QW×3 times
Tumor volume (mm³) Mean ± SEM
Days
ES0204 PDX
G1, B, 3 mg/kg, i.v, QW×3 times
G2, A, 3 mg/kg, i.v, QW×3 times
Tumor volume (mm³) Mean ± SEM
Days
ES0218 PDX
G1, B, 5 mg/kg, i.v, QW×3 times
G2, A, 5 mg/kg, i.v, QW×3 times
Tumor volume (mm³) Mean ± SEM
Days
ES0219 PDX
G1, B, 5 mg/kg, i.v, QW×3 times
G2, A, 5 mg/kg, i.v, QW×3 times
Tumor volume (mm³) Mean ± SEM
Days

Figure 1

**Phase IIa**

| Screening period | Treatment period<br>The planned total enrollment is approximately 80-120 patients | Follow-up period |
|---|---|---|
| Study population:<br>• Advanced solid tumors (mainly esophageal cancer)<br>• At least 1 target lesion<br>• ECOG PS 0-1<br>• Expected survival ≥ 12 weeks<br>• Good organ function | Cohort 1: Patients with unresectable locally advanced, recurrent, or metastatic esophageal squamous cell carcinoma who have progressed after at least one prior line of therapy or are intolerant to such therapy<br>• Anti-B7-H3 antibody-drug conjugate, 10 mg/kg, administered intravenously Q3W (approximately 40 patients)<br>Cohort 2: Patients with unresectable locally advanced, recurrent, or metastatic esophageal adenocarcinoma or adenocarcinoma of the esophagogastric junction who have progressed after at least one prior line of therapy or are intolerant to such therapy<br>• Anti-B7-H3 antibody-drug conjugate, 10 mg/kg, administered intravenously Q3W (approximately 20 patients)<br>Cohort 3: Patients with other advanced solid tumors for whom no standard treatment is available or who have failed standard therapy<br>• Anti-B7-H3 antibody-drug conjugate, 10 mg/kg, administered intravenously Q3W (approximately 20 patients) | **Primary endpoint:**<br>• ORR as assessed by the investigator according to RECIST 1.1<br>**Secondary endpoints:**<br>• Efficacy (DCR, RoR, PFS, and OS)<br>• Safety<br>• PK characteristics<br>• Immunogenicity<br>**Exploratory endpoints:**<br>• Correlation between B7-H3 expression and efficacy, etc. |

**Phase IIb**

| Screening period | Treatment period<br>The planned total enrollment is approximately 100 patients | Follow-up period |
|---|---|---|
| Study population:<br>• Esophageal squamous cell carcinoma<br>• At least 1 target lesion<br>• ECOG PS 0-1<br>• Expected survival ≥ 12 weeks<br>• Adequate organ function | Patients with unresectable locally advanced, recurrent, or metastatic esophageal squamous cell carcinoma who have progressed after or are intolerant to at least first-line treatment<br>• Anti-B7-H3 antibody-drug conjugate, 10 mg/kg, administered intravenously Q3W (the estimated total enrollment is approximately 100 patients; the final sample size will be determined by statistical calculations before the Phase II trial) | **Primary endpoint:**<br>• ORR as assessed by the investigator according to RECIST 1.1<br>**Secondary endpoints:**<br>• Efficacy (DCR, RoR, PFS, and OS)<br>• Safety<br>• PK characteristics<br>• Immunogenicity<br>**Exploratory endpoints:**<br>• Correlation between B7-H3 expression and efficacy, etc. |

Figure 2

**INTERNATIONAL SEARCH REPORT**

International application No. **PCT/CN2024/127218**

**A. CLASSIFICATION OF SUBJECT MATTER**

A61K47/68(2017.01)i; A61K39/395(2006.01)i; A61K31/4745(2006.01)i; C07K16/28(2006.01)i; A61P35/00(2006.01)i

According to International Patent Classification (IPC) or to both national classification and IPC

**B. FIELDS SEARCHED**

Minimum documentation searched (classification system followed by classification symbols)

IPC: A61K C07K A61P

Documentation searched other than minimum documentation to the extent that such documents are included in the fields searched

Electronic data base consulted during the international search (name of data base and, where practicable, search terms used)

CNTXT; ENTXTC; VCN; CJFD; DWPI; ENTXT; CNKI; WFNPL; NPTXT; NPABS; Web of Science; STNext; NCBI; EMBL; 中国专利生物序列检索系统, China Patent Biological Sequence Search System; 读秀, Duxiu: 依喜替康, 艾沙替康, 依沙替康, 食管, 食道, 癌, 肿瘤, 鳞, Esophageal squamous, cancer, tumor, esophagus, B7H3, h1702DS, B7-H3, B7 homolog 3 protein, CD276, B7-CD28, DX 8951, Exatecan, DX-8951a, SEQ ID NO: 1-10的序列检索, search for SEQ ID NOs: 1-10

**C. DOCUMENTS CONSIDERED TO BE RELEVANT**

| Category* | Citation of document, with indication, where appropriate, of the relevant passages | Relevant to claim No. |
|---|---|---|
| X | WO 2020063673 A1 (JIANGSU HENGRUI MEDICINE CO., LTD. et al.) 02 April 2020 (2020-04-02)<br>description, page 15, paragraph 3 from the bottom, page 26, last paragraph, and page 27, paragraph 1, embodiments 11 and 12, and test examples 7 and 9 | 23-32 |
| X | WO 2020063676 A1 (JIANGSU HENGRUI MEDICINE CO., LTD. et al.) 02 April 2020 (2020-04-02)<br>claims 1-46 | 23-32 |
| A | 曹娜娜等 (CAO, Nana et al.). "共刺激分子B7-H3在人食管鳞癌中的表达及临床意义 (Expression of Costimulatory Molecule B7-H3 in Human Esophageal Squamous Cell Carcinoma and Clinical Significance)"<br>*肿瘤防治研究 (Cancer Research on Prevention and Treatment),*<br>Vol. 41, No. (12), 31 December 2014 (2014-12-31), pp. 1300-1303<br>abstract | 23-32 |

☐ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **21 January 2025** | **23 January 2025** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

**INTERNATIONAL SEARCH REPORT**

International application No.
**PCT/CN2024/127218**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☐ forming part of the international application as filed.
   b. ☐ furnished subsequent to the international filing date for the purposes of international search (Rule 13*ter*.1(a)),
      ☐ accompanied by a statement to the effect that the sequence listing does not go beyond the disclosure in the international application as filed.

2. ☑ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

**INTERNATIONAL SEARCH REPORT**

International application No.
**PCT/CN2024/127218**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **1-22**
because they relate to subject matter not required to be searched by this Authority, namely:

The method involved in claims 1-22 is a disease treatment method that is practiced on a living human or animal body for the direct purpose of disease treatment, which belongs to methods for treatment of the human or animal body, and falls within the cases set out in PCT Rule 39.1(iv) for which an international search is not required.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**INTERNATIONAL SEARCH REPORT**
**Information on patent family members**

International application No.
**PCT/CN2024/127218**

| Patent document cited in search report | Publication date (day/month/year) | Patent family member(s) | Publication date (day/month/year) |
|---|---|---|---|
| WO 2020063673 A1 | 02 April 2020 | MX 2021003446 A | 15 June 2021 |
| | | JP 2022512568 A | 07 February 2022 |
| | | JP 7408646 B2 | 05 January 2024 |
| | | EP 3854816 A1 | 28 July 2021 |
| | | EP 3854816 A4 | 07 September 2022 |
| | | ZA 202102696 B | 25 October 2023 |
| | | TW 202028242 A | 01 August 2020 |
| | | TWI 846736 B | 01 July 2024 |
| | | CA 3114474 A1 | 02 April 2020 |
| | | AU 2019351427 A1 | 15 April 2021 |
| | | US 2021347894 A1 | 11 November 2021 |
| | | BR 112021004829 A2 | 08 June 2021 |
| | | KR 20210068457 A | 09 June 2021 |
| WO 2020063676 A1 | 02 April 2020 | ZA 202102544 B | 25 October 2023 |
| | | CA 3114137 A1 | 02 April 2020 |
| | | JP 2022511351 A | 31 January 2022 |
| | | JP 7558156 B2 | 30 September 2024 |
| | | BR 112021004656 A2 | 01 June 2021 |
| | | EP 3858386 A1 | 04 August 2021 |
| | | EP 3858386 A4 | 12 October 2022 |
| | | KR 20210066833 A | 07 June 2021 |
| | | MX 2021003382 A | 27 May 2021 |
| | | US 2021353764 A1 | 18 November 2021 |
| | | AU 2019349207 A1 | 08 April 2021 |
| | | TW 202027795 A | 01 August 2020 |
| | | TWI 826543 B | 21 December 2023 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2020063673 A


### Non-patent literature cited in the description


- **CHOTHIA et al.** *Nature*, 1989, vol. 342, 877-883
- **AL-LAZIKANI et al.** Standard conformations for the canonical structures of immunoglobulins. *Journal of Molecular Biology*, 1997, vol. 273, 927-948
- **KABAT et al.** Sequences of Proteins of Immunological Interest. U.S. Department of Health and Human Services, 1987
- **WARD et al.** *Nature*, 1989, vol. 341, 544-546
- **CHAPOVAL AI** ; **NI J** ; **LAU JS et al.** B7-H3: A costimulatory molecule for T cell activation and IFN-gamma production. *Nat Immunol.*, 2001, vol. 2 (3), 269-274
- **WANG L** ; **KANG FB** ; **SHAN BE**. B7-H3-mediated tumor immunology: Friend or foe?. *Int J Cancer*, 2014, vol. 134 (12), 2764-2771
- **ZHANG G** ; **HOU J** ; **SHI J et al.** Soluble CD276 (B7-H3) is released from monocytes, dendritic cells and activated T cells and is detectable in normal human serum. *Immunology*, 2008, vol. 123 (4), 538-546
- **LI G** ; **QUAN YC** ; **CHE FY et al.** B7-H3 in tumors: friend or foe for tumor immunity?. *Cancer Chemother Pharmacol*, 2018, vol. 81 (2), 245-253
- **WANG L** ; **ZHANG Q** ; **CHEN W et al.** B7-H3 is overexpressed in patients suffering osteosarcoma and associated with tumor aggressiveness and metastasis. *PLoS One*, 05 August 2013, vol. 8 (8), e70689
- **PICARDA E** ; **OHAEGBULAM KC** ; **ZANG X**. Molecular Pathways: Targeting B7-H3 (CD276) for Human Cancer Immunotherapy. *Clin Cancer Res.*, 2016, vol. 22 (14), 3425-3431
- **HU J** ; **JIANG C** ; **ZHENG M et al.** Overexpression of B7-H3 as an opportunity for targeted therapy in head and neck cancers. *Am J Transl Res*, 15 August 2019, vol. 11 (8), 5183-5196
- **LIN W** ; **XU Y** ; **GAO J et al.** Multi-Omics Data Analyses Identify B7-H3 as a Novel Prognostic Biomarker and Predict Response to Immune Checkpoint Blockade in Head and Neck Squamous Cell Carcinoma. *Front Immunol*, 05 October 2021, vol. 12, 757047
- **ALTAN, M.** ; **PELEKANOU, V.** ; **SCHALPER, K. A.** ; **TOKI, M.** ; **GAULE, P.** ; **SYRIGOS, K.** ; **HERBST, R. S.** ; **RIMM, D. L.** B7-H3 Expression in NSCLC and Its Association with B7-H4, PD-L1 and Tumor-Infiltrating Lymphocytes. *Clinical cancer research: an official journal of the American Association for Cancer Research*, 2017, vol. 23 (17), 5202-5209
- **ZHOU, WT.** ; **JIN, WL**. B7-H3/CD276: An Emerging Cancer Immunotherapy. *Front Immunol*, 2021, vol. 12, 701006
- **MARUKI Y** ; **TAKASHIMA A** ; **MIYAMOTO T et al.** Expression of B7-H3 (CD276) in surgically resected esophageal squamous cell carcinoma. *Journal of Clinical Oncology*, 01 February 2018, vol. 36 (4), 70-70
- **SONG J** ; **SHI W** ; **ZHANG Y** ; **SUN M** ; **LIANG X** ; **ZHENG S**. Epidermal growth factor receptor and B7-H3 expression in esophageal squamous tissues correlate to patient prognosis. *Onco Targets Ther*, 12 October 2016, vol. 9, 6257-6263
- **SIEGEL RL** ; **MILLER KD** ; **FUCHS HE** ; **JEMAL A**. Cancer statistics. *CA Cancer J Clin*, 2022, vol. 72 (1), 7-33
- **MORGAN E** ; **SOERJOMATARAM I** ; **RUMGAY H et al.** The global landscape of esophageal squamous cell carcinoma and esophageal adenocarcinoma incidence and mortality in 2020 and projections to 2040: New estimates from GLOBOCAN 2020. *Gastroenterology*, 04 June 2022
- **THRIFT AP**. Global burden and epidemiology of Barrett oesophagus and oesophageal cancer. *Nat Rev Gastroenterol Hepatol.*, 2021, vol. 18 (6), 432-443
- **SUN JM et al.** Pembrolizumab plus chemotherapy versus chemotherapy alone for first-line treatment of advanced oesophageal cancer (KEYNOTE-590): a randomised, placebo-controlled, phase 3 study [published correction appears. *Lancet*, 2021, vol. 398 (10302), 759-771

- **LUO H** ; **LU J** ; **BAI Y et al.** Effect of Camrelizumab vs Placebo Added to Chemotherapy on Survival and Progression-Free Survival in Patients With Advanced or Metastatic Esophageal Squamous Cell Carcinoma: The ESCORT-1st Randomized Clinical Trial. *JAMA*, 2021, vol. 326 (10), 916-925
- **DOKI Y** ; **AJANI JA** ; **KATO K et al.** Nivolumab Combination Therapy in Advanced Esophageal Squamous-Cell Carcinoma. *N Engl J Med*, 2022, vol. 386 (5), 449-462
- **WANG ZX** ; **CUI C** ; **YAO J et al.** Toripalimab plus chemotherapy in treatmentnaïve, advanced esophageal squamous cell carcinoma (JUPITER-06): A multi-center phase 3 trial. *Cancer Cell*, 2022, vol. 40 (3), 277-288
- **HUANG J** ; **XU J** ; **CHEN Y et al.** Camrelizumab versus investigator's choice of chemotherapy as second-line therapy for advanced or metastatic oesophageal squamous cell carcinoma (ESCORT): a multicentre, randomised, open-label, phase 3 study. *Lancet Oncol*, 2020, vol. 21 (6), 832-842
- **KOJIMA T** ; **SHAH MA** ; **MURO K et al.** Randomized Phase III KEYNOTE-181 Study of Pembrolizumab Versus Chemotherapy in Advanced Esophageal Cancer. *J Clin Oncol*, 2020, vol. 38 (35), 4138-4148